# EUROPEAN PATENT APPLICATION

(11) **EP 2 065 380 A1**
(43) Date of publication of application: **03.06.2009**
(21) Application number: 07016399.3
(22) Date of filing: 22.08.2007
(51) Int. Cl.: C07D 401/12, C07D 213/82, A61K 31/444, A61P 35/00

(54) **Pyridoneamide derivatives as focal adhesion kinase (FAK) inhibitors and their use for the treatment of cancer**

(71) Applicant: F.HOFFMANN-LA ROCHE AG, 4070 Basel (CH)
(72) Inventor: The designation of the inventor has not yet been filed
(74) Representative: Schreiner, Siegfried

(57) **Abstract**

Objects of the present invention are the compounds of formula I 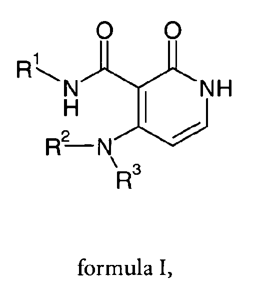 their pharmaceutically acceptable salts, enantiomeric forms, diastereoisomers and racemates, the preparation of the above-mentioned compounds, medicaments containing them and their manufacture, as well as the use of the above-mentioned compounds in the control or prevention of illnesses such as cancer.

## Description

The present invention relates to novel pyridoneamide derivatives, to a process for their manufacture, pharmaceutical compositions containing them and their manufacture as well as the use of these compounds as pharmaceutically active agents.

### Background of the Invention

Protein kinases ("PKs") are enzymes that catalyze the phosphorylation of hydroxy groups on tyrosine, serine and threonine residues of proteins (Hunter, T., Cell 50 (1987) 823-829). The consequences of this seemingly simple activity are staggering; cell growth, differentiation and proliferation, i.e., virtually all aspects of cell life in one way or another depend on PK activity. Furthermore, abnormal PK activity has been related to a host of disorders, ranging from relatively non-life threatening diseases such as psoriasis to extremely virulent diseases such as glioblastoma (brain cancer).

The PKs can be conveniently broken down into two classes, the protein tyrosine kinases (PTKs) and the serine-threonine kinases (STKs).

One of the prime aspects of PTK activity is their involvement with growth factor receptors. Growth factor receptors are cell-surface proteins. When bound by a growth factor ligand, growth factor receptors are converted to an active form which interacts with proteins on the inner surface of a cell membrane. This leads to phosphorylation on tyrosine residues of the receptor and other proteins and to the formation inside the cell of complexes with a variety of cytoplasmic signaling molecules that, in turn, effect numerous cellular responses such as cell division (proliferation), cell differentiation, cell growth, expression of metabolic effects to the extracellular microenvironment, etc. For a more complete discussion, see Schlessinger and Ullrich, Neuron 9 (1992) 303-391, which is incorporated by reference, including any drawings, as if fully set forth herein.

Growth factor receptors with PTK activity are known as receptor tyrosine kinases ("RTKs"). They comprise a large family of transmembrane receptors with diverse biological activity. At present, at least nineteen (19) distinct subfamilies of RTKs have been identified. An example of these is the subfamily designated the "HER" RTKs, which include EGFR (epidermal growth factor receptor), HER2 (human epidermal growth factor receptor 2), HER3 and HER4. These RTKs consist of an extracellular glycosylated ligand binding domain, a transmembrane domain and an intracellular cytoplasmic catalytic domain that can phosphorylate tyrosine residues on proteins.

Another RTK subfamily consists of insulin receptor (IR), insulin-like growth factor I receptor (IGF-1R) and insulin receptor related receptor (IRR). IR and IGF-1R interact with insulin, IGF-I and IGF-II to form a heterotetramer of two entirely extracellular glycosylated α subunits and two β subunits which cross the cell membrane and which contain the tyrosine kinase domain.

Another RTK subfamily is referred to as the platelet derived growth factor receptor ("PDGFR") group, which includes PDGFR alpha, PDGFR beta, colony-stimulating factor 1 receptor (CSF-1R), c-kit and flt-3. These receptors consist of glycosylated extracellular domains composed of 5 immunoglobin-like loops and an intracellular domain wherein the tyrosine kinase domain is interrupted by a kinase inert domain.

Another group which, because of its similarity to the PDGFR subfamily, is sometimes subsumed into the latter group is the fetal liver kinase ("Flk") receptor subfamily. This group, containing extracellulos immunoglobulin loops made up of kinase insert domain- receptor fetal liver kinase-1 (KDR/Flk-1), and fins-like tyrosine kinase 1 (Flt-1 and Flt-4).

A further member of the tyrosine kinase growth factor receptor family is the fibroblast growth factor ("FGF") receptor subgroup. This group consists of four receptors, FGFR1-4, and many ligands. Although there is considerable alternative splicing, generally the receptors consist of a glycosylated extracellular domain containing 3 immunoglobin-like loops and an intracellular domain in which the tyrosine kinase sequence is interrupted by regions of a kinase insert domain.

Still another member of the tyrosine kinase growth factor receptor family is MET, often referred to as c-Met also known as human hepatocyte growth factor receptor tyrosine kinase (hHGFR). c-Met is thought to play a role in primary tumor growth and metastasis.

A more complete listing of the known RTK subfamilies is described in Plowman et al., DN&P 7 (1994) 334-339, which is incorporated by reference, including any drawings, as if fully set forth herein.

In addition to the RTKs, there also exists a family of entirely intracellular PTKs called "non-receptor tyrosine kinases" or "cytoplasmic tyrosine kinases." This latter designation, abbreviated "CTK", will be used herein. CTKs do not contain extracellular and transmembrane domains. At present, over 24 CTKs in 11 subfamilies (Src, Frk, Btk, Csk, Abl, Zap70, Fes, FAK, Jak, LIMK and Ack) have been identified. The Src subfamily appear so far to be the largest group of CTKs and includes Src, Yes, Fyn, Lyn, Lck, Elk, Hck, Fgr, and Yrk. A further important group of CTKs is the Abl family including Abl and Arg. For a more detailed discussion of CTKs, see Bolen, J. B., Oncogene 8 (1993) 2025-2031, which is incorporated by reference, including any drawings, as if fully set forth herein.

The serine/threonine kinases, STKs, like the CTKs, are predominantly intracellular although there are a few receptor kinases of the STK type. STKs are the most common of the cytosolic kinases; i.e., kinases that perform their function in that part of the cytoplasm other than the cytoplasmic organelles and cytoskelton. The cytosol is the region within the cell where much of the cell's intermediary metabolic and biosynthetic activity occurs; e.g., it is in the cytosol that proteins are synthesized on ribosomes. The STKs include CDk2, Raf, the ZC family of kinases, the NEK family of kinases, and BUB1.

Focal adhesion kinase (FAK) is a non-receptor tyrosine kinase involved in integrin-mediated signal transduction pathways. FAK colocalizes with integrins in focal contact sites and FAK activation and its tyrosine phosphorylation have been shown in many cell types to be dependent on integrins binding to their extracellular ligands. Results from several studies support the hypothesis that FAK inhibitors could be useful in cancer treatment. For example, FAK-deficient cells migrate poorly in response to chemotactic signals and overexpression of C-terminal domain of FAK blocks cell spreading as well as chemotactic migration (Sieg, et al., J. Cell Science 112 (1999) 2677-2691; Richardson, A. and Parsons, T., Cell 97 (1997) 221-231); in addition, tumor cells treated with FAK antisense oligonucleotides lost their attachment and underwent apoptosis (Xu, et al, Cell Growth Differ. 4 (1996) 413-418). FAK has been reported to be overexpressed in prostate, breast, thyroid, colon and lung cancers. The level of expression of FAK is directly correlated with tumors demonstrating the most aggressive phenotype.

RTKs, CTKs and STKs have all been implicated in a host of pathogenic conditions including, significantly, cancer. Other pathogenic conditions which have been associated with PTKs include, without limitation, psoriasis, hepatic cirrhosis, diabetes, angiogenesis, fibrosis, restenosis, ocular diseases, rheumatoid arthritis and other inflammatory disorders, immunological disorders such as autoimmune disease, cardiovascular disease such as atherosclerosis and a variety of renal disorders.

With regard to cancer, two of the major hypotheses advanced to explain the excessive cellular proliferation that drives tumor development relate to functions known to be PK regulated. That is, it has been suggested that malignant cell growth results from a breakdown in the mechanisms that control cell division and/or differentiation. It has been shown that the protein products of a number of proto-oncogenes are involved in the signal transduction pathways that regulate cell growth and differentiation. These protein products of proto-oncogenes include the extracellular growth factors, transmembrane growth factor PTK receptors (RTKs), cytoplasmic PTKs (CTKs) and cytosolic STKs, discussed above.

In view of the apparent link between PK-related cellular activities and wide variety of human disorders, it is no surprise that a great deal of effort is being expended in an attempt to identify ways to modulate PK activity. Some of these have been made to identify small molecules which act as PK inhibitors.

WO 02/079192, WO2004/031401, WO2004/063151 and WO2005/021510 relate to benzimidazole pyridone derived kinase inhibitors. WO 01/030758 relates to N-Phenyl-1,2-dihydro-1-methyl-2-oxoquinoline-3-carboxamide derivatives as anticancer agents.

### Summary of the Invention

The present invention relates to indole derivatives of the general formula I, 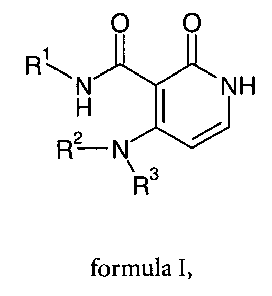 wherein
- R¹: is phenyl, which is unsubstituted or substituted one or several times independently by alkyl, -OH, -O-alkyl, -S-alkyl, -S(O)₂- alkyl, halogen, -NRR', -CH₂-NRR', trifluoromethyl, trifluoromethoxy, heterocyclyl which is unsubstituted or substituted once or twice by alkyl or acetyl, -CH₂-heterocyclyl, - C(O)-NRR', -C(O)-NH-CH₂-phenyl-R", -C(O)-NH-phenyl- R";
- R²: is hydrogen or (C₁-C₃)alkyl;
- R³: is -X-R⁴;
- R⁴: is a) (C₁-C₄)alkyl; b) (C₃-C₆)cycloalkyl; c) a 5 to 7 membered heterocycle, which is unsubstituted or substituted once or twice by alkyl; d) phenyl, wherein the phenyl is unsubstituted or substituted one to three times by halogen, alkyl, -O-CH₃, -N(CH₃)₂, trifluoromethyl or trifluoromethoxy; or e) heteroaryl;
- X: is (C₁-C₄)alkylene, -S(O)₂-, or a single bond, wherein the (C₁- C₄)alkylene is unsubstituted or substituted once or twice by hydroxy, alkyl or halogen;
- R and R': represent independently of each other hydrogen or (C₁-C₃)alkyl;
and all pharmaceutically acceptable salts thereof.

The compounds according to this invention show activity as protein kinase inhibitors. Many diseases are associated with abnormal cellular responses triggered by protein kinase mediated events. These diseases include autoimmune diseases, inflammatory diseases, neurological and neurodegenerative diseases, cancer, cardiovascular diseases, allergies and asthma, Alzheimer's disease or hormone-related diseases. Accordingly, there has been a substantial effort in medicinal chemistry to find protein kinase inhibitors that are effective as therapeutic agents.

The compounds according to this invention in particular show activity as kinase inhibitors, especially as FAK inhibitors.

Objects of the present invention are the compounds of formula I and their tautomers, pharmaceutically acceptable salts, enantiomeric forms, diastereoisomers and racemates, their use as protein kinase inhibitors, in particular as FAK inhibitors, the preparation of the above-mentioned compounds, medicaments or pharmaceutical compositions containing them and their manufacture as well as the use of the above-mentioned compounds in treatment, control or prevention of illnesses, especially of illnesses and disorders as mentioned above like tumors or cancer (e.g. colorectal, breast, lung, prostate, pancreatic, gastric, bladder, ovarian, melanoma, neuroblastoma, cervical, kidney or renal cancers, leukemias or lymphomas) or in the manufacture of corresponding medicaments or pharmaceutical compositions.

### Detailed Description of the Invention

### 1. Definitions:

The term "alkyl" as used herein means a saturated, straight-chain or branched-chain hydrocarbon containing from 1 to 5 carbon atoms, preferably from 1 to 4 carbon atoms, such as methyl, ethyl, n-propyl, isopropyl, n-butyl, 2-butyl, t-butyl, n-pentyl.

The term " (C₁-C₃)alkyl" as used herein means an alkyl as defined above containing from 1 to 3 carbon atoms.

The term "halogen" as used herein means fluorine, chlorine or bromine, preferably fluorine or chlorine and more preferably chlorine.

The term "(C₁-C₄)alkylene" as used herein means a saturated, straight-chain, hydrocarbon containing from 1 to 4 carbon atoms, preferably from 1 to 3 carbon atoms such as methylene, ethylene, trimethylene (1,3-propylene) or tetramethylene (1,4-butylene).

The term "(C₃-C₆) cycloalkyl" means a monocyclic saturated hydrocarbon ring with 3 to 6, ring atoms. Examples of such saturated carbocyclic groups are cyclopropyl, cyclobutyl, cyclopentyl and cyclohexyl, preferably cyclohexyl.

The term "heterocyclyl" as used herein means a saturated, monocyclic ring with 5 to 6 ring atoms which contains up to 3 heteroatoms, preferably 1 or 2 heteroatoms, selected independently from N, O or S and the remaining ring atoms being carbon atoms. Preferably at least one heteroatom of the ring is N and the remaining heteroatoms are selected independently from N, O or S. Examples of such saturated heterocyclic groups pyrrolidinyl, morpholinyl, piperazinyl, piperidyl, oxazolidinyl, thiazolidinyl, and the like, preferably pyrrolidinyl, piperidinyl, morpholino or piperazinyl.

The term "a 5 to 7 membered heterocycle" as used herein means a saturated, monocyclic ring with 5 to 7 ring atoms, preferably with 5 to 6 ringatoms, which contains up to 3 heteroatoms, preferably 1 or 2 heteroatoms, selected independently from N, O or S and the remaining ring atoms being carbon atoms. Preferably at least one heteroatom of the ring is N and the remaining heteroatoms are selected independently from N, O or S. Examples of such saturated 5 to 7 membered heterocycles include pyrrolidinyl, morpholinyl, piperazinyl, piperidyl, oxazolidinyl, thiazolidinyl, and the like, preferably pyrrolyl, piperidinyl, morpholino or piperazinyl, more preferably pyrrolidinyl or piperidinyl.

The term "heteroaryl" as used herein means a monocyclic aromatic ring with with 5 to 6 ring atoms, which contains up to 3 heteroatoms, preferably 1 or 2 heteroatoms, selected independently from N, O or S and the remaining ring atoms being carbon atoms. Examples of such heteroaryl groups include pyrrolyl, imidazolyl, pyrazolyl, triazolyl, tetrazolyl, furanyl, oxazolyl, isoxazolyl, thienyl, thiazolyl, pyridyl, pyrimidyl, pyridazinyl, pyrazinyl, and the like, preferably pyridyl, indolyl or imidazolyl, more preferably pyridyl.

### 2. Detailed Description:

R¹ is phenyl, which is unsubstituted or substituted one or several times, preferably one to three times, independently by alkyl, -OH, -O-alkyl, -S-alkyl, -S(O)₂-alkyl, halogen, preferably chlorine or fluorine, -NRR', -CH₂-NRR', trifluoromethyl, trifluoromethoxy, heterocyclyl which is unsubstituted or substituted once or twice by alkyl or acetyl, -CH₂-heterocyclyl, -C(O)-NRR', -C(O)-NH-CH₂-phenyl-R", -C(O)-NH-phenyl-R"; preferably by alkyl, -OH, -O-CH₃, -S-CH₃, -S(O)₂-CH₃, chlorine or fluorine, -NRR', -CH₂-NRR', trifluoromethyl, pyrrolidinyl, piperidinyl, morpholino, piperazinyl, methyl-piperazinyl, acetyl-piperazinyl, -CH₂-pyrrolyl, -CH₂-piperidinyl, -C(O)-NRR'.

R² is hydrogen or (C₁-C₃)alkyl; preferably hydrogen or methyl, and more preferably hydrogen.

R³ is -X-R⁴.

R⁴ is a) (C₁-C₄)alkyl; preferably (C₁-C₃)alkyl (if X is single bond);
b) (C₃-C₆)cycloalkyl; preferably cyclohexyl (if X is single bond);
c) a 5 to 7 membered heterocycle, which is unsubstituted or substituted once or twice by alkyl; preferably such heterocycle is selected frompyrrolidinyl, piperidinyl or methyl-piperidinyl;
d) phenyl, wherein the phenyl is unsubstituted or substituted one to three times, preferably once, by halogen, alkyl, -O-CH₃, -N(CH₃)₂, trifluoromethyl or trifluoromethoxy; preferably by chlorine, alkyl, -O-CH₃, -N(CH₃)₂, or trifluoromethyl; more preferably once by chlorine;
or e) heteroaryl, preferably selected from pyridyl, indolyl or imidazolyl, more preferably the heteroaryl is pyridyl.

X is (C₁-C₄)alkylene, -S(O)₂-, or a single bond, wherein the (C₁-C₄)alkylene is unsubstituted or substituted once or twice, preferably once, by hydroxy, alkyl or halogen; preferably by hydroxy, alkyl or chlorine , more preferably by hydroxy or methyl.

R and R' represent independently of each other hydrogen or (C₁-C₃)alkyl. Preferably the significance of R and R' is (C₁-C₃)alkyl for -NRR', -CH₂-NRR' and the significance of R and R' is hydrogen for -C(O)-NRR'.

An embodiment of the invention are the compounds according to formula I, characterized in that
- R¹: is phenyl, which is unsubstituted or substituted one to three times, independently by alkyl, -OH, -O-CH₃, -S-CH₃, -S(O)₂- CH₃, chlorine, fluorine, -NRR', -CH₂-NRR', trifluoromethyl, - CH₂-pyrrolyl, -CH₂-piperidinyl, -C(O)-NRR', or heterocyclyl selected from pyrrolidinyl, piperidinyl, morpholino, piperazinyl, methyl-piperazinyl, or acetyl-piperazinyl;
- R²: is hydrogen or methyl; preferably hydrogen;
- R⁴: is a) (C₁-C₃)alkyl; b) cyclohexyl; c) a 5 to 7 membered heterocycle selected from pyrrolidinyl, piperidinyl or methyl-piperidinyl; d) phenyl, wherein the phenyl is unsubstituted or substituted once by chlorine, alkyl, -O-CH₃, -N(CH₃)₂, or trifluoromethyl; or e) heteroaryl selected from pyridyl, indolyl or imidazolyl; and
- X: is (C₁-C₄)alkylene, -S(O)₂-, or a single bond, wherein the (C₁- C₄)alkylene is unsubstituted or substituted once by hydroxy or methyl.

Another embodiment of the invention are the compounds according to formula I, characterized in that
- R⁴: is a) (C₁-C₃)alkyl; or
b) cyclohexyl.

Such compounds, for example, may be selected from the group consisting of:
4-Ethylamino-2-oxo-1,2-dihydro-pyridine-3-carboxylic acid [4-methoxy-3-(4-methyl-piperazin-1-yl)-phenyl]-amide;
4-Isopropylamino-2-oxo-1,2-dihydro-pyridine-3-carboxylic acid [4-methoxy-3-(4-methyl-piperazin-1-yl)-phenyl]-amide; and
4-Cyclohexylamino-2-oxo-1,2-dihydro-pyridine-3-carboxylic acid [4-methoxy-3-(4-methyl-piperazin-1-yl)-phenyl]-amide.

Another embodiment of the invention are the compounds according to formula I, characterized in that
- R⁴: is a 5 to 7 membered heterocycle selected from pyrrolidinyl, piperidinyl or methyl-piperidinyl.

Such compounds, for example, may be selected from the group consisting of:
2-Oxo-4-(2-piperidin-1-yl-ethylamino)-1,2-dihydro-pyridine-3-carboxylic acid [3-(4-methyl-piperazin-1-yl)-phenyl]-amide;
4-(1-Methyl-piperidin-4-ylamino)-2-oxo-1,2-dihydro-pyridine-3-carboxylic acid [4-methoxy-3-(4-methyl-piperazin-1-yl)-phenyl]-amide; and
2-Oxo-4-(2-pyrrolidin-1-yl-ethylamino)-1,2-dihydro-pyridine-3-carboxylic acid [4-methoxy-3-(4-methyl-piperazin-1-yl)-phenyl]-amide.

Another embodiment of the invention are the compounds according to formula I, characterized in that
- R⁴: is phenyl, wherein the phenyl is unsubstituted or substituted once by chlorine, alkyl, -O-CH₃, -N(CH₃)₂, or trifluoromethyl.

Such compounds, for example, may be selected from the group consisting of:
4- [2-(3-Chloro-phenyl)-ethylamino]-2-oxo-1,2-dihydro-pyridine-3-carboxylic acid (3-fluoro-4-morpholin-4-yl-phenyl)-amide;
4-((S)-2-Hydroxy-2-phenyl-ethylamino)-2-oxo-1,2-dihydro-pyridine-3-carboxylic acid (3-fluoro-4-morpholin-4-yl-phenyl)-amide;
4-[(S)-2-(3-Chloro-phenyl)-2-hydroxy-ethylamino]-2-oxo-1,2-dihydro-pyridine-3-carboxylic acid (3-fluoro-4-morpholin-4-yl-phenyl)-amide;
4-((S)-2-Hydroxy-2-phenyl-ethylamino)-2-oxo-1,2-dihydro-pyridine-3-carboxylic acid (3-pyrrolidin-1-yl-phenyl)-amide;
4-[2-(3-Chloro-phenyl)-ethylamino]-2-oxo-1,2-dihydro-pyridine-3-carboxylic acid (3-pyrrolidin-1-yl-phenyl)-amide;
4-[(S)-2-(3-Chloro-phenyl)-2-hydroxy-ethylamino]-2-oxo-1,2-dihydro-pyridine-3-carboxylic acid (4-dimethylamino-phenyl)-amide;
4-[2-(3-Chloro-phenyl)-2-hydroxy-ethylamino]-2-oxo-1,2-dihydro-pyridine-3-carboxylic acid (4-piperidin-1-yl-phenyl)-amide;
4-[(S)-2-(3-Chloro-phenyl)-2-hydroxy-ethylamino]-2-oxo-1,2-dihydro-pyridine-3-carboxylic acid (4-pyrrolidin-1-yl-3-trifluoromethyl-phenyl)-amide;
4-[2-(3-Chloro-phenyl)-ethylamino]-2-oxo-1,2-dihydro-pyridine-3-carboxylic acid (4-pyrrolidin-1-yl-3-trifluoromethyl-phenyl)-amide;
4-[(S)-2-Hydroxy-2-phenyl-ethylamino]-2-oxo-1,2-dihydro-pyridine-3-carboxylic acid (4-pyrrolidin-1-yl-3-trifluoromethyl-phenyl)-amide;
4-[2-(3-Chloro-phenyl)-ethylamino]-2-oxo-1,2-dihydro-pyridine-3-carboxylic acid (3-piperidin-1-yl-phenyl)-amide;
4-[(S)-2-(3-Chloro-phenyl)-2-hydroxy-ethylamino]-2-oxo-1,2-dihydro-pyridine-3-carboxylic acid [4-(4-methyl-piperazin-1-yl)-phenyl]-amide;
4-[(S)-2-Hydroxy-2-phenyl-ethylamino]-2-oxo-1,2-dihydro-pyridine-3-carboxylic acid [4-(4-methyl-piperazin-1-yl)-phenyl]-amide;
4-[(S)-2-(3-Chloro-phenyl)-2-hydroxy-ethylamino]-2-oxo-1,2-dihydro-pyridine-3-carboxylic acid (3-piperidin-1-yl-phenyl)-amide;
4-[(S)-2-Hydroxy-2-phenyl-ethylamino]-2-oxo-1,2-dihydro-pyridine-3-carboxylic acid (3-dimethylamino-phenyl)-amide;
4-[2-(3-Chloro-phenyl)-ethylamino]-2-oxo-1,2-dihydro-pyridine-3-carboxylic acid (3-dimethylamino-phenyl)-amide;
4-[(S)-2-(3-Chloro-phenyl)-2-hydroxy-ethylamino]-2-oxo-1,2-dihydro-pyridine-3-carboxylic acid (3-dimethylamino-phenyl)-amide;
4-[(S)-2-Hydroxy-2-phenyl-ethylamino]-2-oxo-1,2-dihydro-pyridine-3-carboxylic acid (3-piperidin-1-yl-phenyl)-amide;
4-[(S)-2-Hydroxy-2-phenyl-ethylamino]-2-oxo-1,2-dihydro-pyridine-3-carboxylic acid [4-(4-acetyl-piperazin-1-yl)-3-trifluoromethyl-phenyl]-amide;
4-[2-(3-Chloro-phenyl)-ethylamino]-2-oxo-1,2-dihydro-pyridine-3-carboxylic acid [3-fluoro-4-(4-methyl-piperazin-1-yl)-phenyl]-amide;
2-Oxo-4-(2-m-tolyl-ethylamino)-1,2-dihydro-pyridine-3-carboxylic acid [3-(4-methyl-piperazin-1-yl)-phenyl]-amide;
4-[2-(2-Methoxy-phenyl)-ethylamino]-2-oxo-1,2-dihydro-pyridine-3-carboxylic acid [3-(4-methyl-piperazin-1-yl)-phenyl]-amide;
4-[2-(4-Methoxy-phenyl)-ethylamino]-2-oxo-1,2-dihydro-pyridine-3-carboxylic acid [3-(4-methyl-piperazin-1-yl)-phenyl]-amide;
4-[2-(3-Chloro-phenyl)-ethylamino]-2-oxo-1,2-dihydro-pyridine-3-carboxylic acid [4-(4-methyl-piperazin-1-yl)-phenyl]-amide;
4-[(S)-2-Hydroxy-2-phenyl-ethylamino]-2-oxo-1,2-dihydro-pyridine-3-carboxylic acid [3-fluoro-4-(4-methyl-piperazin-1-yl)-phenyl]-amide;
4-[(S)-2-(3-Chloro-phenyl)-2-hydroxy-ethylamino]-2-oxo-1,2-dihydro-pyridine-3-carboxylic acid [4-(4-acetyl-piperazin-1-yl)-3-trifluoromethyl-phenyl]-amide;
4-[(S)-2-Hydroxy-2-phenyl-ethylamino]-2-oxo-1,2-dihydro-pyridine-3-carboxylic acid (4-dimethylamino-phenyl)-amide;
4-[2-(3-Chloro-phenyl)-ethylamino]-2-oxo-1,2-dihydro-pyridine-3-carboxylic acid (4-dimethylamino-phenyl)-amide;
4-[(S)-2-(3-Chloro-phenyl)-2-hydroxy-ethylamino]-2-oxo-1,2-dihydro-pyridine-3-carboxylic acid (2,6-dimethyl-phenyl)-amide;
4-[(S)-2-(3-Chloro-phenyl)-2-hydroxy-ethylamino]-2-oxo-1,2-dihydro-pyridine-3-carboxylic acid [4-methoxy-3-(4-methyl-piperazin-1-yl)-phenyl]-amide;
4-[(S)-2-(3-Chloro-phenyl)-2-hydroxy-ethylamino]-2-oxo-1,2-dihydro-pyridine-3-carboxylic acid (2,6-dimethoxy-phenyl)-amide;
4-[(S)-2-(3-Chloro-phenyl)-2-hydroxy-ethylamino]-2-oxo-1,2-dihydro-pyridine-3-carboxylic acid (2-chloro-6-methyl-phenyl)-amide;
4-[(S)-2-(3-Chloro-phenyl)-2-hydroxy-ethylamino]-2-oxo-1,2-dihydro-pyridine-3-carboxylic acid (2-methoxy-6-methyl-phenyl)-amide;
4-[(S)-2-(3-Chloro-phenyl)-2-hydroxy-ethylamino]-2-oxo-1,2-dihydro-pyridine-3-carboxylic acid (3-morpholin-4-yl-phenyl)-amide;
4-[(S)-2-(3-Chloro-phenyl)-2-hydroxy-ethylamino]-2-oxo-1,2-dihydro-pyridine-3-carboxylic acid (4-morpholin-4-yl-phenyl)-amide;
4-[(S)-2-(3-Chloro-phenyl)-2-hydroxy-ethylamino]-2-oxo-1,2-dihydro-pyridine-3-carboxylic acid (4-carbamoyl-2,6-dimethyl-phenyl)-amide;
4-[(S)-2-(3-Chloro-phenyl)-2-hydroxy-ethylamino]-2-oxo-1,2-dihydro-pyridine-3-carboxylic acid (3-methanesulfonyl-phenyl)-amide;
4-[(S)-2-(3-Chloro-phenyl)-2-hydroxy-ethylamino]-2-oxo-1,2-dihydro-pyridine-3-carboxylic acid (3-methylsulfanyl-phenyl)-amide;
4-((S)-2-Hydroxy-2-phenyl-ethylamino)-2-oxo-1,2-dihydro-pyridine-3-carboxylic acid (3-piperidin-1-ylmethyl-phenyl)-amide;
4-((S)-2-Hydroxy-2-phenyl-ethylamino)-2-oxo-1,2-dihydro-pyridine-3-carboxylic acid (3-pyrrolidin-1-ylmethyl-phenyl)-amide;
4- [(2-Hydroxy-2-phenyl-ethyl)-methyl-amino]-2-oxo-1,2-dihydro-pyridine-3-carboxylic acid [3-(4-methyl-piperazin-1-yl)-phenyl]-amide;
4-(2-Hydroxy-2-phenyl-ethylamino)-2-oxo-1,2-dihydro-pyridine-3-carboxylic acid [3-(4-methyl-piperazin-1-yl)-phenyl]-amide;
2-Oxo-4-phenethylamino-1,2-dihydro-pyridine-3-carboxylic acid [3-(4-methyl-piperazin-1-yl)-phenyl]-amide;
4-(Methyl-phenethyl-amino)-2-oxo-1,2-dihydro-pyridine-3-carboxylic acid [3-(4-methyl-piperazin-1-yl)-phenyl]-amide;
4-[2-(3-Methoxy-phenyl)-ethylamino]-2-oxo-1,2-dihydro-pyridine-3-carboxylic acid [3-(4-methyl-piperazin-1-yl)-phenyl]-amide;
4-[(2-Hydroxy-2-phenyl-ethyl)-methyl-amino]-2-oxo-1,2-dihydro-pyridine-3-carboxylic acid [4-(4-methyl-piperazin-1-yl)-phenyl]-amide;
2-Oxo-4-phenethylamino-1,2-dihydro-pyridine-3-carboxylic acid (3-morpholin-4-yl-phenyl)-amide;
2-Oxo-4-(2-phenyl-propylamino)-1,2-dihydro-pyridine-3-carboxylic acid (3-morpholin-4-yl-phenyl)-amide;
2-Oxo-4-phenethylamino-1,2-dihydro-pyridine-3-carboxylic acid [4-methoxy-3-(4-methyl-piperazin-1-yl)-phenyl]-amide;
2-Oxo-4-(2-phenyl-propylamino)-1,2-dihydro-pyridine-3-carboxylic acid [4-methoxy-3-(4-methyl-piperazin-1-yl)-phenyl]-amide;
4-[2-(3-Chloro-phenyl)-ethylamino]-2-oxo-1,2-dihydro-pyridine-3-carboxylic acid [4-methoxy-3-(4-methyl-piperazin-1-yl)-phenyl]-amide;
4-Benzenesulfonylamino-2-oxo-1,2-dihydro-pyridine-3-carboxylic acid [4-methoxy-3-(4-methyl-piperazin-1-yl)-phenyl]-amide;
2-Oxo-4-phenethylamino-1,2-dihydro-pyridine-3-carboxylic acid (3-piperidin-1-ylmethyl-phenyl)-amide;
2-Oxo-4-(2-phenyl-propylamino)-1,2-dihydro-pyridine-3-carboxylic acid (3-piperidin-1-ylmethyl-phenyl)-amide;
2-Oxo-4-(2-phenyl-propylamino)-1,2-dihydro-pyridine-3-carboxylic acid (3-pyrrolidin-1-ylmethyl-phenyl)-amide;
2-Oxo-4-phenethylamino-1,2-dihydro-pyridine-3-carboxylic acid (3-pyrrolidin-1-ylmethyl-phenyl)-amide;
2-Oxo-4-phenethylamino-1,2-dihydro-pyridine-3-carboxylic acid (3-diethylaminomethyl-4-hydroxy-phenyl)-amide;
4-(Methyl-phenethyl-amino)-2-oxo-1,2-dihydro-pyridine-3-carboxylic acid [4-methoxy-3-(4-methyl-piperazin-1-yl)-phenyl]-amide;
2-Oxo-4-phenethylamino-1,2-dihydro-pyridine-3-carboxylic acid [4-(4-methyl-piperazin-1-yl)-phenyl]-amide; and
2-Oxo-4-(2-phenyl-propylamino)-1,2-dihydro-pyridine-3-carboxylic acid [4-(4-methyl-piperazin-1-yl)-phenyl]-amide.

Another embodiment of the invention are the compounds according to formula I, characterized in that
- R⁴: is heteroaryl selected from pyridyl, indolyl or imidazolyl.

Such compounds, for example, may be selected from the group consisting of:
2-Oxo-4-[(pyridin-2-ylmethyl)-amino]-1,2-dihydro-pyridine-3-carboxylic acid (3-fluoro-4-morpholin-4-yl-phenyl)-amide;
2-Oxo-4-[(pyridin-2-ylmethyl)-amino]-1,2-dihydro-pyridine-3-carboxylic acid (3-pyrrolidin-1-yl-phenyl)-amide;
2-Oxo-4-[(pyridin-2-ylmethyl)-amino]-1,2-dihydro-pyridine-3-carboxylic acid (4-pyrrolidin-1-yl-3-trifluoromethyl-phenyl)-amide;
2-Oxo-4-[(pyridin-2-ylmethyl)-amino]-1,2-dihydro-pyridine-3-carboxylic acid [3-fluoro-4-(4-methyl-piperazin-1-yl)-phenyl]-amide;
2-Oxo-4-[(pyridin-2-ylmethyl)-amino]-1,2-dihydro-pyridine-3-carboxylic acid (3-piperidin-1-yl-phenyl)-amide;
2-Oxo-4-[(pyridin-2-ylmethyl)-amino]-1,2-dihydro-pyridine-3-carboxylic acid [4-(4-methyl-piperazin-1-yl)-phenyl]-amide;
2-Oxo-4-[(pyridin-2-ylmethyl)-amino]-1,2-dihydro-pyridine-3-carboxylic acid (3-dimethylamino-phenyl)-amide;
4-[2-(3H-Imidazol-4-yl)-ethylamino]-2-oxo-1,2-dihydro-pyridine-3-carboxylic acid [3-(4-methyl-piperazin-1-yl)-phenyl]-amide;
4-[2-(1H-Indol-3-yl)-ethylamino]-2-oxo-1,2-dihydro-pyridine-3-carboxylic acid [3-(4-methyl-piperazin-1-yl)-phenyl]-amide;
2-Oxo-4-[(pyridin-2-ylmethyl)-amino]-1,2-dihydro-pyridine-3-carboxylic acid (4-dimethylamino-phenyl)-amide;
2-Oxo-4-[(pyridin-2-ylmethyl)-amino]-1,2-dihydro-pyridine-3-carboxylic acid (3-morpholin-4-yl-phenyl)-amide;
2-Oxo-4-[(pyridin-2-ylmethyl)-amino]-1,2-dihydro-pyridine-3-carboxylic acid (2,6-dimethyl-phenyl)-amide;
2-Oxo-4-[(pyridin-2-ylmethyl)-amino]-1,2-dihydro-pyridine-3-carboxylic acid [4-methoxy-3-(4-methyl-piperazin-1-yl)-phenyl]-amide;
2-Oxo-4-[(pyridin-2-ylmethyl)-amino]-1,2-dihydro-pyridine-3-carboxylic acid (2-methoxy-6-methyl-phenyl)-amide;
2-Oxo-4-[(pyridin-2-ylmethyl)-amino]-1,2-dihydro-pyridine-3-carboxylic acid (2,6-dimethoxy-phenyl)-amide;
2-Oxo-4-[(pyridin-2-ylmethyl)-amino]-1,2-dihydro-pyridine-3-carboxylic acid (2-chloro-6-methyl-phenyl)-amide;
2-Oxo-4-(2-pyridin-2-yl-ethylamino)-1,2-dihydro-pyridine-3-carboxylic acid (3-morpholin-4-yl-phenyl)-amide;
2-Oxo-4-(2-pyridin-2-yl-ethylamino)-1,2-dihydro-pyridine-3-carboxylic acid [4-methoxy-3-(4-methyl-piperazin-1-yl)-phenyl]-amide;
2-Oxo-4-[(pyridin-2-ylmethyl)-amino]-1,2-dihydro-pyridine-3-carboxylic acid (4-carbamoyl-2,6-dimethyl-phenyl)-amide;
2-Oxo-4-[(pyridin-2-ylmethyl)-amino]-1,2-dihydro-pyridine-3-carboxylic acid (4-morpholin-4-yl-phenyl)-amide;
2-Oxo-4-[(pyridin-2-ylmethyl)-amino]-1,2-dihydro-pyridine-3-carboxylic acid (3-methylsulfanyl-phenyl)-amide;
2-Oxo-4-(2-pyridin-3-yl-ethylamino)-1,2-dihydro-pyridine-3-carboxylic acid [4-methoxy-3-(4-methyl-piperazin-1-yl)-phenyl]-amide; and
2-Oxo-4-(2-pyridin-4-yl-ethylamino)-1,2-dihydro-pyridine-3-carboxylic acid [4-methoxy-3-(4-methyl-piperazin-1-yl)-phenyl]-amide.

Another embodiment is a process for the preparation of the compounds of formula I comprising the steps of
a) reacting a compound of formula III, 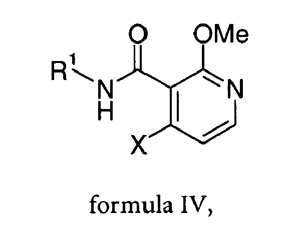 wherein R¹ has the meaning of formula I as defined above and X represents a halogen selected from chlorine, bromine and iodine,
   with a compound of formula VII 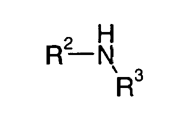 wherein R¹ and R² have the meaning of formula I as defined above,
b) cleavage of the methoxy group of the pyridine residue,
   wherein such cleavage of the methoxy group of the pyridine can also be performed before step a)
to give the compounds of formula I, 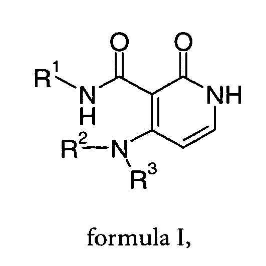 wherein R¹, R² and R³ have the meaning of formula I as defined above.

The pyridinone amide derivatives compounds of formula I, or a pharmaceutically acceptable salt thereof, which are subject of the present invention, may be prepared by any process known to be applicable to the preparation of chemically-related compounds. Such processes, when used to prepare a compound of the formula I, or a pharmaceutically-acceptable salt thereof, are illustrated by the following representative scheme 1 and examples in which, unless otherwise stated, R¹, R² and R² have the significance given herein before for formula I. Necessary starting materials are either commercially available or they may be obtained by standard procedures of organic chemistry. The preparation of such starting materials is described within the accompanying examples or in the literature cited below with respect to scheme 1. Alternatively necessary starting materials are obtainable by analogous procedures to those illustrated which are within the ordinary skill of an organic chemist.

The pyridinone amides of the general formula I bearing substituted amino-side chains can be prepared on a straightforward synthesis route starting from anilines of formula II and pyridine carboxylates of formula III. The intermediate nicotinic acid amide of formula IV can be further modified as described below to afford the desired pyridinoneamides of the general formula I.

Amines and in particular anilines of formula II wherein R¹ has the meaning defined herein before are commercially available or can be prepared by standard methods of organic chemistry which are familiar to those skilled in the art. One of these standard methods is the reduction of the corresponding nitrobenzene bearing the appropriate substitution pattern to the corresponding aniline. Other methods include, but are not limited to, the introduction and/or the transformation of substituents at the phenyl ring of N-protected anilines by standard methods of organic chemistry. Protection and deprotection strategies are also known to those skilled in the art.

Pyridine carboxylates of formula III wherein A is either OH (free acids), Cl (acid chlorides), hydrogen (aldehydes), OAlk (alkyl carboxylates ) or hydroxybenzotriazole (activated esters) and X represents a halogen selected from chlorine, bromine and iodine, can be prepared according to literature procedures.

Pyridine aldehydes of formula III (A is H and X represents a halogen selected from chlorine, bromine and iodine) can for example be prepared according to procedures described in WO 95/29917 or Gabarda, et al., Tetrahedron 2002, 58 (32), 6329.

Pyridine carboxylates of formula III (A is OH and X represents a halogen selected from chlorine, bromine and iodine) can for example be prepared according to procedures described in W02004063151.

Alkyl pyridine carboxylates of formula III (A is e.g. OMe and X represents a halogen selected from chlorine, bromine and iodine) can for example be prepared according to procedures described in W02004063151.

The intermediate nicotinic acid amide of formula IV wherein R¹ has the meaning defined herein before can be further reacted to compounds of formula I by two different ways (scheme 1):
a) Cleavage of the methoxy group of the pyridine residue by e.g. treatment with a strong inorganic acid in an appropriate solvent (for instance hydrochloric acid in 1,4-dioxane) to form the pyridine-2-one intermediates of formula V and subsequent introduction of the side chain of compound I by substitution with suitable nucleophiles of formula VII wherein R² and R³ have the meaning defined herein before at elevated temperatures to yield compounds of formula I. A similar strategy for the synthesis of aminopyridin-2-ones is described in e.g. WO2002079192, US20040044203, WO2004031401, WO2004063151, and Wittman, et al., J. Med. Chem. 48(18) (2005) 5639-5643.
b) Introduction of the side chain of compound I by substitution with suitable nucleophiles of formula VII wherein R² and R³ have the meaning defined herein before applying e.g. Pd-catalyzed cross-coupling reactions or Cucatalyzed aminations (based on general procedures for C-N- and C-O-bond formation with aromatic iodides described in e.g. Kwong, et al., Org. Lett. 4(4) (2002) 581-584, Wolter, et al., Org. Lett. 4(6) (2002) 973, Kwong, et al., Org. Lett. 5(6) (2003) 793-796, and Org. Lett. 5 (23) (2003) 4373) to form the methoxypyridine intermediates VI and subsequent cleavage of the methoxy group of the pyridine residue by e.g. treatment with a strong inorganic acid in an appropriate solvent (for instance hydrochloric acid in 1,4-dioxane) to form the compounds of formula I.

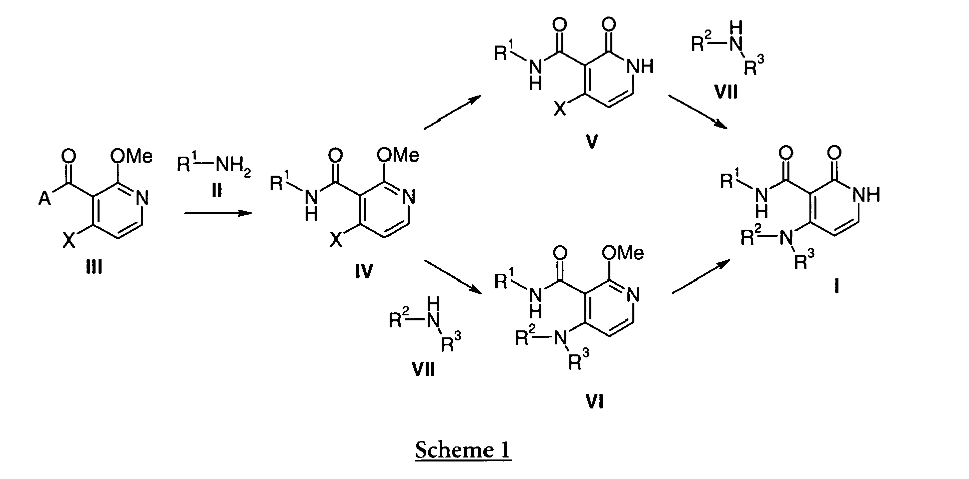

Certain substituents on the groups R¹, R² and R³ may not be inert to the conditions of the synthesis sequences described above and may require protection by standard protecting groups known in the art. For instance, an amino or hydroxyl group may be protected as an acetyl or tert.-butoxycarbonyl derivative. Alternatively, some substituents may be derived from others at the end of the reaction sequence. For instance, a compound of formula I may be synthesized bearing a nitro-, an ethoxycarbonyl, an ether, a sulfonic acid substituent on the groups R¹, R² and R³, which substituents are finally converted to an amino- (e.g. by reduction of a nitro group or cleavage of a suitable amino protective group (e.g. removal of a Boc group with TFA)), alkylamino- (e.g. by reductive amination of an amino group), dialkylamino- (e.g. by alkylation of an amino group, reduction of an appropriate acylamino group with lithium aluminum hydride or Eschweiler-Clarke reaction with an appropriate amino or alkylamino group), acylamino- (by amide formation from an amino group e.g. with appropriate acyl halides or with appropriate carboxylic acids after their activation with CDI, EDC etc.), alkylsulfonylamino (e.g. by reaction of an amino group with sulfonyl chlorides), arylsulfonylamino substituent (e.g. by reaction of an amino group with sulfonyl chlorides), hydroxyl-(by cleavage of a suitable hydroxy protective group (e.g. hydrogenolytic removal of a benzyl ether or oxidative cleavage of a p-methoxy benzyl ether), ether- (e.g. by Williamson's ether synthesis from a hydroxyl group) or to a carboxamide substituent (e.g. by amide formation from a carboxylic acid group with appropriate amines after activation of the carboxylic acid group with CDI, EDC etc. or conversion to an acyl chloride), or to a sulfonamide substituent by standard procedures.

Pharmaceutical compositions containing a compound of the present invention or a pharmaceutically acceptable salt thereof and a pharmaceutically acceptable carrier are an object of the present invention, as is a process for their production, which comprises bringing one or more compounds of the present invention and/or pharmaceutically acceptable salts and, if desired, one or more other therapeutically valuable substances into a galenical administration form together with one or more pharmaceutically acceptable carriers.

In accordance with the invention the compounds of the present invention as well as their pharmaceutically acceptable salts are useful in the control or prevention of illnesses. Based on their protein kinase activity and their antiproliferative activity, said compounds are useful for the treatment of diseases such as cancer in humans or animals and for the production of corresponding pharmaceutical compositions. The dosage depends on various factors such as manner of administration, species, age and/or individual state of health.

An embodiment of the invention is a pharmaceutical composition, containing one or more compounds according to formula I as active ingredients, together with pharmaceutically acceptable carriers.

Another embodiment of the invention is a pharmaceutical composition, containing one or more compounds according to formula I as active ingredients, for the inhibition of tumor growth.

Another embodiment of the invention is a pharmaceutical composition, containing one or more compounds according to formula I as active ingredients, for the treatment of cancer.

Another embodiment of the invention is a pharmaceutical composition containing one or more compounds of formula I as active ingredients together with pharmaceutically acceptable carriers for the treatment of colorectal, breast, lung, prostate, pancreatic, gastric, bladder, ovarian, melanoma, neuroblastoma, cervical, kidney or renal cancers, leukemias or lymphomas.

Another embodiment of the invention is the use of a compound according to formula I, for the manufacture of corresponding pharmaceutical compositions for the inhibition of tumor growth.

Another embodiment of the invention is the use of a compound according to formula I, for the manufacture of corresponding pharmaceutical compositions for the treatment of colorectal, breast, lung, prostate, pancreatic, gastric, bladder, ovarian, melanoma, neuroblastoma, cervical, kidney or renal cancers, leukemias or lymphomas.

Another embodiment of the invention is the use of the compounds of formula I as anti-proliferating agents.

Another embodiment of the invention is the use of one or more compounds of formula I for the treatment of cancer.

Another embodiment of the invention is a method of treating cancer comprising administering to a person in need thereof a therapeutically effective amount of a compound of formula I.

Another embodiment of the invention is a method of treating cancer comprising administering to a person in need thereof a therapeutically effective amount of a compound of formula I, wherein the cancer is colorectal cancer, breast cancer, lung cancer, prostate cancer, pancreatic cancer, gastric cancer, bladder cancer, ovarian cancer, melanoma, neuroblastoma, cervical cancer, kidney cancer or renal cancer, leukemia, or lymphoma.

The compounds according to the present invention may exist in the form of their pharmaceutically acceptable salts. The term "pharmaceutically acceptable salt" refers to conventional acid-addition salts that retain the biological effectiveness and properties of the compounds of formula I and are formed from suitable non-toxic organic or inorganic acids. Sample acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, naphthalenesulfonic acid, naphthalenedisulfonic acid, methanesulfonic acid, ethanesulfonic acid, citric acid, ascorbic acid and the like. The chemical modification of a pharmaceutical compound (i.e. a drug) into a salt is a technique well known to pharmaceutical chemists to obtain improved physical and chemical stability, hygroscopicity, flowability and solubility of compounds (see, e.g., Stahl, P.H. and Wermuth, G. (editors), Handbook of Pharmaceutical Salts, Verlag Helvetica Chimica Acta (VHCA), Zürich (2002), or Bastin, R.J., et al., Organic Proc. Res. Dev. 4 (2000) 427-435).

The compounds of formula I can contain one or several chiral centers and can then be present in a racemic or in an optically active form. The racemates can be separated according to known methods into the enantiomers. For instance, diastereomeric salts which can be separated by crystallization are formed from the racemic mixtures by reaction with an optically active acid such as e.g. D- or L-camphorsulfonic acid. Alternatively separation of the enantiomers can also be achieved by using chromatography on chiral HPLC-phases (HPLC: High Performance Liquid Chromatography) which are commercially available.

### Pharmacological activity

The compounds of formula I and their pharmaceutically acceptable salts possess valuable pharmacological properties. It has been found that said compounds show activity as FAK inhibitors and also show anti-proliferative activity. Consequently the compounds of the present invention are useful in the therapy and/or prevention of proliferative diseases and illnesses with known over-expression of kinases.

### IC50 determination for inhibitors of FAK:

### Assay principle

The ELISA-type assay is based on a biotinylated substrate which is phosphorylated by the kinase ("autophosphorylation"). The phosphorylated peptide is detected by antiphosphotyrosine monoclonal antibody conjugated with horse radish peroxidase. As FAK proteinthe FAK Kinase Domain ( KD) from baculo Sf9 was used. Substrate peptide (FAK-397): N-terminal labelled with biotin: Biotin-GO-VSVSETDDYAEIIDEED (MW2538). Anti-phosphotyrosine monoclonal antibody PT66 peroxidase conjugate (Sigma Cat. No.5964.)

Kinase activity was determined in 50 mM Hepes pH 7.3 containing 0.01% Triton X-100 (Roche Diagnostics Cat. No. 14942521), 1mM TCEP (Pierce Cat. No. 20490), 1.0 mM MgCl₂, 10 µM Na3VO4, 10 µM adenosine triphosphate.

### Enzyme reaction in polypropylene plate:

| **Step** | **Action** | **Volume (**µ**l)** |
|---|---|---|
| 1 | add buffer, MnCl2, ATP | 15 |
| 2 | add diluted compound | 15 |
| 3 | start reaction with enzyme | 15 |
| 4 | incubate for 30 min | |
| 5 | stop with EDTA | 15 |

### Wash/Detection:

- Transfer of 50 µl aliquot
- Wash 4 times with 100 µl PBS (Roche Cat. No. 1666789)
- Addition of 50 µl 1:3000 diluted anti-phospho-tyrosine PT66 Mab (12 mg/ml) in 0.5% bovine serum albumin(BSA)/0.05% Tween20/PBS for 60 min
- Wash 6 times with 100 µl PBS

Addition of 50 µl 2,2'-azino-di-(3 ethylbenzthiazoline) sulfonic acid reagent(ABTS reagent = ABTS Buffer (Roche Cat. No. 10473300)/ABTS tablets (Roche Cat. No. 14942521 )- 2 tablets (50 mg) are dissolved in 125 ml ABTS Buffer ) reagent for 60 min. Absorption at 405 nm/0.1 sec was measured. The inhibitory avtivity (IC50) using a non-linear curve fit (XLfit software (ID Business Solution Ltd., Guilford, Surrey, UK))

**Table 1**

| **Example No.** | **IC50 FAK inhibition [µM]** |
|---|---|
| 1 | 0.143 |
| 2d | 0.093 |
| 6aa | 0.216 |
| 2a, 2b, 2c, 2f,2g, 2h, 2q, 2s,2x, 3, 4a, 4c, 4e, 4i, 4m, 4n, 4°, 4q, 4s, 4v, 4y, 4aa, 4ab, 4ac, 4ae, 6b, 6g, 6j, 61, 6m, 6n, 6o, 6p, 6r, 6t, 6y, 6z, 6aa, 6ae | 0.001-2.000 |

### Antiproliferative activity:

The activity of the present compounds as antiproliferative agents is demonstrated by the following biological assay:

### CellTiter-Glo^{™} assay in HCT 116 cells:

The CellTiter-GloTM Luminescent Cell Viability Assay (Promega) is a homogeneous method of determining the number of viable cells in culture based on quantitation of the ATP present, which signals the presence of metabolically active cells.

HCT 116 cells (human colon carcinoma, ATCC-No. CCl-247) were cultivated in RPMI 1640 medium with GlutaMAX™ I (Invitrogen, Cat-No. 61870-010), 5 % Fetal Calf Serum (FCS, Sigma Cat-No. F4135 (FBS)); 100Units/ml penicillin/100µg/ml streptomycin (= Pen/Strep from Invitrogen Cat. No. 15140). For the assay the cells were seeded in 384 well plates, 1000 cells per well, in the same medium. The next day the test compounds were added in various concentrations ranging from 30 µM to 0.0015 µM (10 concentrations, 1:3 diluted). After 5 days the CellTiter-Glo^{™} assay was done according to the instructions of the manufacturer (CellTiter-Glo^{™} Luminescent Cell Viability Assay, from Promega). In brief: the cell-plate was equilibrated to room temperature for approximately 30 minutes and than the CellTiter-Glo^{™} reagent was added. The contents were carefully mixed for 15 minutes to induce cell lysis. After 45 minutes the luminescent signal was measured in Victor 2, (scanning multiwell spectrophotometer, Wallac).

### Details:

**1st. day:**
- Medium: RPMI 1640 with GlutaMAX^{™} I (Invitrogen, Cat-Nr. 61870), 5 % FCS (Sigma Cat.-No. F4135), Pen/Strep (Invitrogen, Cat No. 15140).
- HCT116 (ATCC-No. CCl-247): 1000 cells in 60 µl per well of 384 well plate (Greiner 781098, µClear-plate white)
- After seeding incubate plates 24 h at 37°C, 5% CO₂

### 2nd. day : Induction (Treatment with compounds, 10 concentrations):

In order to achieve a final concentration of 30 µM as highest concentration 3,5 µl of 10 mM compound stock solution were added directly to 163 µl media. Then step e) of the dilution procedure described below, was followed.

In order to achieve the second highest to the lowest concentrations, a serial dilution with dilution steps of 1:3 was followed according to the procedure (a -e) as described here below:
a) for the second highest concentration add 10 µl of 10 mM stock solution of compound to 20 µl dimethylsulfoxide (DMSO)
b) dilute 8x 1:3 (always 10 µl to 20 µl DMSO) in this DMSO dilution row (results in 9 wells with concentrations from 3333,3 µM to 0.51 µM)
c) dilute each concentration 1: 47,6 (3,5 µl compound dilution to 163 µl media)
d) add 10 µl of every concentration to 60 µl media in the cell plate resulting in final concentration of DMSO : 0.3 % in every well and resulting in 10 final concentration of compounds ranging from 30 µM to 0.0015 µM.

- Each compound is tested in triplicate.
- Incubate 120 h (5 days) at 37°C, 5% CO₂

### Analysis:

- Add 30 µl CellTiter-Glo^{™} Reagent (prepared from CellTiter-Glo^{™} Buffer and CellTiter-Glo^{™} Substrate (lyophilized) purchased from Promega) per well,
- shake 15 minutes at room temperature
- incubate further 45 minutes at room temperature without shaking

### Measurement:

- Victor 2 scanning multiwell spectrophotometer (Wallac), Luminescence mode (0.5 sec/read, 477 nm)
- Determine IC50 using a non-linear curve fit (XLfit software (ID Business Solution Ltd., Guilford, Surrey, UK))

With all compounds a significant inhibition of HCT 116 cell viability was detected, which is exemplified by the compounds shown in Table 1.

**Table 2**

| **Example No.** | **IC50 HCT 116 [µM]** |
|---|---|
| 1 | 7.462 |
| 2m | 3.691 |
| 4d | 1.249 |
| 2a, 2e, 2j, 2k, 2m,2n, 2o,2p, 2s, 2w, 3, 4a, 4b, 4c, 4i, 4m, 4p, 4q, 4v, 4y, 4aa, 4ab, 4ae, 6g, 6i, 6j, 6m, 6o, 6r, 6s, 6t, 6u, 6v, 6w, 6ad, 6ae | 0.500-15.000 |

The compounds according to this invention and their pharmaceutically acceptable salts can be used as medicaments, e.g. in the form of pharmaceutical compositions. The pharmaceutical compositions can be administered orally, e.g. in the form of tablets, coated tablets, dragées, hard and soft gelatine capsules, solutions, emulsions or suspensions. The administration can, however, also be effected rectally, e.g. in the form of suppositories, or parenterally, e.g. in the form of injection solutions.

The above-mentioned pharmaceutical compositions can be obtained by processing the compounds according to this invention with pharmaceutically inert, inorganic or organic carriers. Lactose, corn starch or derivatives thereof, talc, stearic acids or it's salts and the like can be used, for example, as such carriers for tablets, coated tablets, dragées and hard gelatine capsules. Suitable carriers for soft gelatine capsules are, for example, vegetable oils, waxes, fats, semi-solid and liquid polyols and the like. Depending on the nature of the active substance no carriers are, however, usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical compositions can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

A pharmaceutical compositions comprise e.g. the following:

### a) Tablet Formulation (Wet Granulation):

| **Item** | **Ingredients** | **Mg/tablet** | | | |
|---|---|---|---|---|---|
| 1. | Compound of formula I | 5 | 25 | 100 | 500 |
| 2. | Lactose Anhydrous DTG (direct tabletting grade) | 125 | 105 | 30 | 150 |
| 3. | Sta-Rx 1500 (pre-gelatinized starch powder) | 6 | 6 | 6 | 30 |
| 4. | Microcrystalline Cellulose | 30 | 30 | 30 | 150 |
| 5. | Magnesium Stearate | 1 | 1 | 1 | 1 |
| | Total | 167 | 167 | 167 | 831 |

### Manufacturing Procedure:

1. Mix items 1, 2, 3 and 4 and granulate with purified water.
2. Dry the granules at 50°C.
3. Pass the granules through suitable milling equipment.
4. Add item 5 and mix for three minutes; compress on a suitable press.

### b) Capsule Formulation:

| **Item** | **Ingredients** | **Mg/capsule** | | | |
|---|---|---|---|---|---|
| 1. | Compound of formula I | 5 | 25 | 100 | 500 |
| 2. | Hydrous Lactose | 159 | 123 | 148 | --- |
| 3. | Corn Starch | 25 | 35 | 0 | 70 |
| 4. | Talc | 10 | 15 | 10 | 25 |
| 5. | Magnesium Stearate | 1 | 2 | 2 | 5 |
| | Total | 200 | 200 | 300 | 600 |

### Manufacturing Procedure:

1. Mix items 1, 2 and 3 in a suitable mixer for 30 minutes.
2. Add items 4 and 5 and mix for 3 minutes.
3. Fill into a suitable capsule.

### c) Micro suspension

1. Weigh 4.0 g glass beads in custom made tube GL 25, 4 cm (the beads fill half of the tube).
2. Add 50 mg compound, disperse with spatulum and vortex.
3. Add 2 ml gelatin solution (weight beads: gelatin solution = 2:1) and vortex.
4. Cap and wrap in aluminum foil for light protection.
5. Prepare a counter balance for the mill.
6. Mill for 4 hours, 20/s in a Retsch mill (for some substances up to 24 hours at 30/s).
7. Extract suspension from beads with two layers of filter (100 µm) on a filter holder, coupled to a recipient vial by centrifugation at 400 g for 2 min.
8. Move extract to measuring cylinder.
9. Repeat washing with small volumes(here 1 ml steps) until final volume is reached or extract is clear.
10. Fill up to final volume with gelatin and homogenize.

The following examples are provided to aid the understanding of the present invention, the true scope of which is set forth in the appended claims. It is understood that modifications can be made in the procedures set forth without departing from the spirit of the invention.

### Experimental procedures

### Precursor 1

### 4-Iodo-2-methoxy-pyridine-3-carboxaldehyde

A solution of 2-methoxypyridine (2.18 g, 20 mmol) in 100 ml THF is cooled to -78 °C and t-BuLi (13 ml of 1.7 M solution) is added which results in a color shift of the solution from clear to yellow/orange. Stirring at same temperature is continued for 30 min and at -60 °C for another 30 min. After cooling down to -78 °C, N-(2-Dimethylaminoethyl)-N-methylformimidine (3.08 ml, 22 mmol) is added within a few minutes. Stirring is continued for another 30 min at -78 °C and 45 min at -20 °C. The reaction mixture is now cooled to -40 °C, n-BuLi (15.6 ml of a 1.6 M solution) is added and stirring at that temperature is continued for 90-120 min. After cooling the reaction mixture to -78 °C, a pre-cooled solution of iodine (10.1 g, 40 mmol) in 100 ml THF is added via syringe until de-colorization stops to occur. Stirring at -78 °C is continued for another 30 min, and the reaction mixture is allowed to warm up to 0°C before the mixture is quenched with 150 ml of saturated Na2S2O3-solution and 100 ml of water. The formed precipitate is dissolving and the solution is loosing its color slowly during stirring overnight at RT. Purification is accomplished by phase separation and extraction of the aqueous phase with diethylether. The combined organic phases are washed with 1N aqueous HCl, saturated aqueous sodium bicarbonate and brine. After drying and evaporation of solvent, 3.5 g of raw product is furnished. The product is purified by flash chromatography using a heptan/ethyl acetate (9:1) eluent yielding 1.0 g (19%) of the desired product. Thoroughly dried glass ware is needed as well as inert gas atmosphere has to be applied for all reactions.

### Precursor 2

### 4-Iodo-2-methoxy-nicotinic acid

2-Methyl-2-butane (1.0ml), sodium dihydrogenphosphate (1.0g, 7.4mmol), water (7ml) and sodium chlorite (0.6g, 6.6mmol) were added to a stirred solution of 4-iodo-2-methoxy-pyridine-3-carbaldehyde (0.74g, 2.9mmol) in *tert*-butanol (10ml) at 0°C. After 10 minutes the reaction was poured into an aqueous solution of formic acid (1M, 50ml) and extracted with ethyl acetate (50ml). The organic layer was extracted with NaOH (1M, 50ml) and the aqueous layer acidified to pH 3 with HCl (conc.). The acidic layer was extracted with ethyl acetate (3 x 30ml) and the organic layer dried (MgSO₄) and concentrated to give the desired product as a yellow solid (0.43g, 54%).

### General Reaction Procedure for Amide Coupling

### N-(3-Fluoro-4-morpholin-4-yl-phenyl)-4-iodo-2-methoxy-nicotinamide

Thionyl chloride (0.27ml, 2.7mmol) and a catalytic amount of DMF was added to a stirred suspension of 4-iodo-2-methoxy-nicotinic acid (0.4g, 1.4mmol) in DCM (10ml) and the reaction was heated to 45°C. After 1 hour the reaction was cooled to room temperature and concentrated under reduced pressure to give the crude acid chloride.

The above crude acid chloride (0.4g, 1.28mmol) in DCM (2.5ml) was added to a stirred solution of 3-fluoro-4-morpholin-4-yl-phenylamine (0.337g, 1.70mmol) and triethylamine (1.0ml, 7.2mmol) in DCM (10ml) and the reaction stirred at room temperature. After 20 hours the reaction was diluted with DCM (20ml) and water (10ml) was added. The layers were separated and the organic layer dried (MgSO₄) and concentrated under reduced pressure to give the desired product as a crude brown solid. Further purification can be accomplished by trituration with diisopropylether. (0.46g, 72% yield); Tr = 1.67 min, *m*/*z* (ES⁺) (M+H)⁺ 458.

### 4-Chloro-2-oxo-1,2-dihydro-pyridine-3-carboxylic acid (3-fluoro-4-morpholin-4-yl-phenyl)-amide; 4-Iodo-2-oxo-1,2-dihydro-pyridine-3-carboxylic acid (3-fluoro-4-morpholin-4-yl-phenyl)-amide

N-(3-Fluoro-4-morpholin-4-yl-phenyl)-4-iodo-2-methoxy-nicotinamide was suspended in 4M HCl dioxane (20ml) and heated to 110°C. After 4 hours the reaction was cooled to room temperature and concentrated under reduced pressure to give the crude product as a 7:3 ratio of the chloro : iodo products (0.46g); Tr = 1.46 min, *m*/*z* (ES⁺) (M+H)⁺ 352 & 354; Tr = 1.50 min, m/z (ES⁺) (M+H)⁺ 444.

According to the above described method for the formation of a pyridoneamide of general formula V wherein R¹ has the meaning defined herein before, all other pyridoneamide derivatives of formula V wherein R¹ has the meaning defined herein before have been prepared following a two-step procedure starting from the corresponding anilines of formula II wherein R¹ has the meaning defined herein before via intermediate nicotineamides of formula IV. Further transformation to the final pyridoneamides of formula I was accomplished according to methods A or B.

### General Reaction Procedure for Amination of Methoxypyridines

### 2-Methoxy-N-(3-morpholin-4-yl-phenyl)-4- [(pyridin-2-ylmethyl)-amino]-nicotinamide

Under inert gas atmosphere, 4-Iodo-2-methoxy-N-(3-morpholin-4-yl-phenyl)-nicotinamide (150 mg, 0.34 mmol), CuI (3.25 mg, 0.017 mmol), N,N-diethyl-2-hydroxy-benzamide (13.2 mg, 0.068 mmol) and K3PO4 ( 145 mg, 0.68 mmol) are mixed and evaporated and flushed with argon in a sealed tube. Dry DMF (1 ml) and 2-(aminomethyl)-pyridine (53 µl, 0.51 mmol) are added and the sealed tube is heated for approx. 20h at 90°C. The reaction mixture is treated with 10 ml ethyl acetate, 10 ml H2O and ca 250 µl conc. NH3. Phases are separated, extracted, washed, dried, and the solvent is evaporated. The product is purified by LCMS affording 54 mg (38 %) of a waxy solid.

According to the above described method for the formation of a nicotinamide of general formula VI wherein R¹, R² and R³ have the meaning defined herein before, all other nicotinamide derivatives of formula VI wherein R¹, R² and R³ have the meaning defined herein before have been prepared following a two-step procedure starting from the corresponding anilines of formula II wherein R¹ has the meaning defined herein before via intermediate nicotineamides of formula IV. Further transformation to the final pyridoneamides of formula I was accomplished according to method C.

### General Reaction Procedures for Synthesis of Final Pyridin-2-ones

### Example 1

### Method A

### 2-Oxo-4-[(pyridin-2-ylmethyl)-amino]-1,2-dihydro-pyridine-3-carboxylic acid (3-fluoro-4-morpholin-4-yl-phenyl)-amide

The mixture of 4-chloro-2-oxo-1,2-dihydro-pyridine-3-carboxylic acid (3-fluoro-4-morpholin-4-yl-phenyl)-amide and 4-iodo-2-oxo-1,2-dihydro-pyridine-3-carboxylic acid (3-fluoro-4-morpholin-4-yl-phenyl)-amide (0.115g) obtained above was added to a stirred solution of 2-(aminomethyl)pyridine (0.053g, 0.49mmol) and triethylamine (0.23ml, 1.6mmol) in acetonitrile (6ml) and the reaction was heated to 70°C. After 20 hours the reaction was cooled to room temperature and concentrated under reduced pressure. The crude solid was triturated with diethyl ether and then methanol to give the desired product as an off-white solid. (0.071g; 55%); Tr = 1.55 min, m/z (ES+) (M+H)⁺ 424; 1H NMR (400 MHz, DMSO-d6) δ ppm 13.10 (1 H, s), 11.29 (1 H, br s), 11.05 (1 H, t), 8.59 (1 H, d), 7.85 - 7.77 (1 H, m), 7.69 (1 H, dd), 7.42 - 7.35 (2 H, m), 7.32 (1 H, dd), 7.14 (1 H, dd), 7.00 (1 H, t), 6.06 (1 H, d), 4.68 (2 H, d), 3.82 - 3.63 (4 H, m), 3.06 - 2.88 (4 H, m)

### Example 2

According to Method A described for Example 1 the following examples have been prepared:

| **Example** | **Systematic name** | **NMR (400 MHz, d6-DMSO):** δ **=** | **MS (ES+): *m*/*z*** |
|---|---|---|---|
| **2a** | 4-[2-(3-Chlorophenyl)-ethylamino]-2-oxo-1,2-dihydropyridine-3-carboxylic acid (3-fluoro-4-morpholin-4-yl-phenyl)-amide | 13.04 (1 H, s), 11.21 (1 H, br. s.), 10.56 (1 H, t), 7.68 (1 H, dd), 7.22 (1 H, s), 7.35 - 7.32 (2 H, m), 7.29 - 7.26 (2 H, m), 7.10 (1 H, dd), 6.98 (1 H, t), 6.12 (1 H, d), 3.73 - 3.71 (4 H, m), 3.53 (2 H, q), 2.96 - 2.93 (4 H, m), 2.90 (2 H, t) | 471 & 473 |
| **2b** | 4-((S)-2-Hydroxy-2-phenyl-ethylamino)-2-oxo-1,2-dihydropyridine-3-carboxylic acid (3-fluoro-4-morpholin-4-yl-phenyl)-amide | 13.09 (1 H, s), 11.07 (1 H, br. s.), 10.77 (1 H, t), 7.68 (1 H, dd), 7.45 (2 H, d), 7.39 - 7.24 (4 H, m), 7.13 (1 H, dd), 7.00 (1 H, t), 6.08 (1 H, d), 5.79 (1 H, d), 4.82 - 4.78 (1 H, m), 3.75 - 3.72 (4 H, m), 3.54 - 3.49 (1 H, m), 3.40-3.36 (1 H, m), 2.97 - 2.95 (4 H, m) | 453 |
| **2c** | 4-[(S)-2-(3-Chlorophenyl)-2-hydroxy-ethylamino] -2-oxo-1,2-dihydro-pyridine-3-carboxylic acid (3-fluoro-4-morpholin-4-yl-phenyl)-amide | 13.07 (1 H, s), 11.16 (1 H, br. s.), 10.74 (1 H, t), 7.70 (1 H, dd), 7.51 (1 H, s), 7.41-7.29 (4 H, m), 7.11 (1 H, dd), 6.99 (1 H, t), 6.10 (1 H, d), 5.91 (1 H, d), 4.85 - 4.81 (1 H, m), 3.74 - 3.72 (4 H, m), 3.56 - 3.51 (1 H, m), 3.39 - 3.30 (1 H, m), 2.96 - 2.94 (4 H, m) | 487 & 489 |
| **2d** | 4-((S)-2-Hydroxy-2-phenyl-ethylamino)-2-oxo-1,2-dihydro-pyridine-3-carboxylic acid (3-pyrrolidin-1-yl-phenyl)-amide | 12.92 (1 H, s), 11.10 (1 H, br. s.), 10.85 (1 H, t, 7.45 (2 H, d), 7.35 (2 H, t), 7.32 - 7.23 (2 H, m), 7.08 (1 H, t), 6.84 - 6.79 (2 H, m), 6.25 (1 H, d), 6.08 (1 H, d), 5.76 (1 H, d), 4.81 - 4.77 (1 H, m), 3.48 - 3.46 (1 H, m), 3.33 (1 H, s), 3.23 - 3.20 (4 H, m), 1.97 - 1.94 (4 H, m) | 419 |
| **2e** | 4-[2-(3-Chlorophenyl)-ethylamino]-2-oxo-1,2-dihydro-pyridine-3-carboxylic acid (3-pyrrolidin-1-yl-phenyl)-amide | 12.88 (1 H, s), 11.14 (1 H, br. s.), 10.67 (1 H, t),7.42 (1 H, s), 7.35 - 7.31 (2 H, m), 7.29 - 7.26 (2 H, m), 7.05 (1 H, t), 6.87 (1 H, s), 6.74 (1 H, d), 6.23 (1 H, dd), 6.12 (1 H, d), 3.56 - 3.51 (2 H, m), 3.22 - 3.19 (4 H, m), 2.90 (2 H, t), 1.96 - 1.93 (4 H, m) | 437 & 439 |
| **2f** | 2-Oxo-4-[(pyridin-2-ylmethyl)-amino]-1,2-dihydro-pyridine-3-carboxylic acid (3-pyrrolidin-1-yl-phenyl)-amide | 12.93 (1 H, s), 11.22 (1 H, d), 11.11 (1 H, t), 8.57 (1 H, d), 7.80 (1 H, td), 7.37 - 7.29 (3 H, m), 7.07 (1 H, t), 6.85 - 6.84 (1 H, m), 6.81 (1 H, d), 6.24 (1 H, dd), 6.01 (1 H, dd), 4.67 (2 H, d), 3.22 - 3.19 (4 H, m), 1.96 - 1.92 (4 H, m) | 390 |
| **2g** | 4-[(S)-2-(3-Chlorophenyl)-2-hydroxy-ethylamino] -2-oxo-1,2-dihydro-pyridine-3-carboxylic acid (4-dimethylamino-phenyl)-amide | 13.01 (s, 1H), 11.14 (m, 1H), 10.88 (t, 1H), 7.51 (s, 1H), 7.41-7.36 (m, 5H), 7.33 (d, 1H), 7.27 (t, 1H), 6.72 (d, 1H), 6.07 (d, 1H), 5.87 (d, 1H), 4.83 (m, 1H), 3.42 (m, 2H), 2.86 (s, 6H) | 427 & 429 |
| **2h** | 4-[2-(3-Chlorophenyl)-2-hydroxy-ethylamino] -2-oxo-1,2-dihydro-pyridine-3-carboxylic acid (4-piperidin-1-yl-phenyl)-amide | 12.98 (s, 1H), 11.04 (d, 1H), 10.85 (t, 1H), 7.51 (s, 1H), 7.43-7.37 (m, 4H), 7.33 (d, 1H), 7.27 (t, 1H), 6.89 (d, 2H), 6.07 (d, 1H), 5.89 (d, 1H), 4.83 (m, 1H), 3.45 (m, 2H), 3.07 (m, 4H), 1.63 (m, 4H), 1.52, (m, 2H) | 467 & 469 |
| **2i** | 4-[(S)-2-(3-Chlorophenyl)-2-hydroxy-ethylamino]-2-oxo-1,2-dihydro-pyridine-3-carboxylic acid (4-pyrrolidin-1-yl-3-trifluoromethyl-phenyl)-amide | 13.08 (1 H, s), 11.18 (1 H, br. s.), 10.74 (1 H, t), 8.05 (1 H, d), 7.54 - 7.51 (2 H, m), 7.41 - 7.29 (4 H, m), 7.19 (1 H, d), 6.10 (1 H, d), 5.91 (1 H, d), 4.85 - 4.81 (1 H, m), 3.57 - 3.51(2H,m),3.17-3.15(4H,m), 1.89 - 1.86 (4 H, m) | 521 & 523 |
| **2j** | 2-Oxo-4-[(pyridin-2-ylmethyl)-amino]-1,2-dihydro-pyridine-3-carboxylic acid (4-pyrrolidin-1-yl-3-trifluoromethyl-phenyl)-amide | 13.10 (1 H, s), 11.28 (1 H, d), 11.02 (1 H, t), 8.57 (1 H, d), 8.05 (1 H, d), 7.79 (1 H, td), 7.54 (1 H, dd), 7.37 - 7.29 (3 H, m), 7.19 (1 H, d), 6.03 (1 H, d), 4.67 (2 H, d), 3.18 - 3.15 (4 H, m), 1.89 - 1.86 (4 H, m) | 458 |
| **2k** | 2-Oxo-4-[(pyridin-2-ylmethyl)-amino]-1,2-dihydro-pyridine-3-carboxylic acid [3-fluoro-4-(4-methyl-piperazin-1-yl)-phenyl]-amide | 13.06 (1 H, s), 11.27 (1 H, br. s.), 11.03 (1 H, t), 8.59 - 8.54 (1 H, m), 7.79 (1 H, td), 7.65 (2 H, dd), 7.40 - 7.27 (3 H, m), 7.10 (1 H, dd), 6.97 (1 H, t), 6.03 (1 H, d), 4.66 (2 H, d), 3.00 - 2.88 (4 H, m), 2.46 - 2.40 (4 H, m), 2.21 (3 H, s) | 437 |
| **2l** | 4-[2-(3-Chlorophenyl)-ethylamino] -2-oxo-1,2-dihydro-pyridine-3-carboxylic acid (4-pyrrolidin-1-yl-3-trifluoromethyl-phenyl)-amide | 13.06 (1 H, s), 11.21 (1 H, br. s.), 10.56 (1 H, t), 8.05 (1 H, d), 7.51 (1 H, dd), 7.42 (1 H, s), 7.35 - 7.26 (4 H, m), 7.18 (1 H, d), 6.14 (1 H, d), 3.53 (2 H, q), 3.17 - 3.14 (4 H, m), 2.90 (2 H, t), 1.89 - 1.86 (4 H, m) | 505 & 507 |
| **2m** | 4-[(S)-2-Hydroxy-2-phenyl-ethylamino] -2-oxo-1,2-dihydro-pyridine-3-carboxylic acid (4-pyrrolidin-1-yl-3-trifluoromethyl-phenyl)-amide | 13.09 (1 H, s), 11.16 (1 H, br. s.), 10.75 (1 H, t), 8.03 (1 H, d), 7.53 (1 H, dd), 7.44 (2 H, d), 7.36 - 7.19 (6 H, m), 6.08 (1 H, d), 5.77 (1 H, d), 4.80 - 4.78 (1 H, m), 3.54 - 3.48 (1 H, m), 3.18 - 3.15 (4 H, m), 1.89 - 1.86 (4 H, m) | 487 |
| **2n** | 4-[2-(3-Chlorophenyl)-ethylamino]-2-oxo-1,2-dihydro- pyridine-3-carboxylic acid (3-piperidin-1-yl-phenyl)-amide | 12.99 (1 H, s), 11.23 (1 H, br. s.), 10.70 (1 H, t), 7.49 (1 H, s), 7.43 - 7.31 (5 H, m), 7.16 (1 H, t), 6.92 (1 H, d), 6.68 (1 H, d), 6.19 (1 H, d), 3.65 - 3.56 (2 H, m), 3.24 - 3.12 (4 H, m), 2.97 (2 H, t), 1.74 - 1.64 (4 H, m), 1.63 - 1.51 (2 H, m) | 451 & 453 |
| **2o** | 2-Oxo-4-[(pyridin-2-ylmethyl)-amino]-1,2-dihydro-pyridine-3-carboxylic acid (3-piperidin-1-yl-phenyl)-amide | 13.04 (1 H, d), 11.07 (1 H, br. s.), 8.57 (1 H, d), 7.78 (1 H, td), 7.37 - 7.29 (3 H, m), 7.24 (1 H, s), 7.12 (1 H, t), 6.93 (1 H, d), 6.62 (1 H, dd), 6.01 (1 H, d), 4.66 (2 H, d), 3.12 - 3.09 (4 H, m), 1.61 - 1.60 (4 H, m), 1.53 - 1.52 (2 H, m) | 404 |
| **2p** | 4-[(S)-2-(3-Chlorophenyl)-2-hydroxy-ethylamino] -2-oxo-1,2-dihydro-pyridine-3-carboxylic acid [4-(4-methyl-piperazin-1-yl)-phenyl] -amide | 12.81 (1 H, s), 11.16 (1 H, d), 10.85 (1 H, t), 7.51 (1 H, s), 7.45 - 7.27 (6 H, m), 6.89 (2 H, d), 6.07 (1 H, d), 5.93 (1 H, d), 4.83 - 4.79 (1 H, m), 3.54 - 3.49 (1 H, m) 3.08 - 3.05 (4 H, m), 2.45 - 2.43 (4 H, m), 2.21 (3 H, s) | 482 & 484 |
| **2q** | 4-[(S)-2-Hydroxy-2-phenyl-ethylamino]-2-oxo-1,2-dihydro-pyridine-3-carboxylic acid [4-(4-methyl-piperazin-1-yl)-phenyl] -amide | 12.81 (1 H, s), 11.15 (1 H, d), 10.86 (1 H, t), 7.43 (4 H, d), 7.34 (2 H, t), 7.29 - 7.25 (2 H, m), 6.89 (2 H, d), 6.05 (1 H, d), 5.79 (1 H, d), 4.79 - 4.76 (1 H, m), 3.51 - 3.46 (1 H, m), 3.08 - 3.05 (4 H, m), 2.45 - 2.43 (4 H, m), 2.21 (3 H, s) | 448 |
| **2r** | 4-[(S)-2-(3-Chlorophenyl)-2-hydroxy-ethylamino]-2-oxo-1,2-dihydro-pyridine-3-carboxylic acid (3-piperidin-1-yl-phenyl)-amide | 13.03 (1 H, br. s.), 11.16 (1 H, d), 10.78 (1 H, t), 7.51 (1 H, s), 7.39 - 7.28 (4 H, m), 7.18 (2 H, br. s.), 7.01 (1 H, br. s.), 6.70 (1 H, br. s.), 6.10 (1 H, d), 5.89 (1 H, br. s.), 4.84 (1 H, dd), 3.57 - 3.51 (1 H, m) 3.18 (4 H, br s), 1.67 (4 H, br s), 1.56 (2 H, br s) | 467 & 469 |
| **2s** | 2-Oxo-4-[(pyridin-2-ylmethyl)-amino]-1,2-dihydro-pyridine-3-carboxylic acid [4-(4-methyl-piperazin-1-yl)-phenyl]-amide | 12.76 (1 H, s), 11.13 (1 H, t), 10.29 (1H, br. s), 8.57 (1H, d), 7.81 (1 H, t), 7.44 (2H, d), 7.28 (1 H, d), 7.32 (2 H, m), 6.89 (2 H, d), 6.07 (1 H, d), 4.65 (2 H, d), 3.09 (4 H, m), 2.50 (4 H, m), 2.24 (3 H, s) | 419 |
| **2t** | 4-[(S)-2-Hydroxy-2-phenyl-ethylamino]-2-oxo-1,2-dihydro-pyridine-3-carboxylic acid (3-dimethylamino-phenyl)-amide | 12.86 (1 H, s), 11.04 (1 H, d), 10.79 (1 H, t), 7.44 - 7.26 (6 H, m), 7.12 (1 H, t), 7.03 (1 H, s), 6.84 (1 H, d), 6.48 (1 H, d), 6.08 (1 H, d), 4.80 (1 H, m), 4.14 (1 H, m),3.39 (2 H, m), 2.90 (6 H, s) | 393 |
| **2u** | 4-[2-(3-Chloro-phenyl)-ethylamino]-2-oxo-1,2-dihydro-pyridine-3-carboxylic acid (3-dimethylamino-phenyl)-amide | 12.86 (1 H, s), 11.09 (1 H, d), 10.62 (1 H, t), 7.40 (1 H, s), 7.30 (2 H, m), 7.28 (2 H, m), 7.11 (1 H, t), 7.06 (1 H, s), 6.85 (1 H, d), 6.47 (1 H, m), 6.13 (1 H, d), 3.53 (2 H, m), 2.91 (8 H, m) | 411 & 413 |
| **2v** | 2-Oxo-4-[(pyridin-2-ylmethyl)-amino]-1,2-dihydro-pyridine-3-carboxylic acid (3-dimethylamino-phenyl)-amide | 12.90 (1 H, s), 11.16 (1 H, d), 11.10 (1 H, m), 8.57 (1 H, d), 7.81 (1 H, m), 7.39 (1 H, d), 7.33 (2 H, m), 7.12 (1 H, t), 7.02 (1 H, s), 6.90 (1 H, d), 6.46 (1 H, m), 6.06 (1 H, d), 4.67 (2 H, d), 2.90 (6 H, s) | 364 |
| **2w** | 4-[(S)-2-(3-Chloro-phenyl)-2-hydroxy-ethylamino]-2-oxo-1,2-dihydro-pyridine-3-carboxylic acid (3-dimethylamino-phenyl)-amide | 12.84 (1 H, s), 11.04 (1 H, d), 10.77 (1 H, t), 7.50 (1 H, s), 7.37 (2 H, m), 7.30 (1 H, d), 7.28 (1 H, t), 7.10 (1 H, t), 7.00 (1 H, s), 6.86 (1 H, d), 6.44 (1 H, m), 6.08 (1 H, d), 4.83 (1 H, br. s), 4.14 (1 H, m), 3.41 ( 2 H, m), 2.89 (1 H, s) | 427 & 429 |
| **2x** | 4-[(S)-2-Hydroxy-2-phenyl-ethylamino]-2-oxo-1,2-dihydro-pyridine-3-carboxylic acid (3-piperidin-1-yl-phenyl)-amide | 13.17 (1 H, s), 11.11 (1 H, d), 10.73 (1 H, t), 7.69 (1 H, br. s), 7.45 (2 H, d), 7.37 - 7.28 (6 H, m), 7.02 (1 H, br. s), 6.10 (1 H, d), 4.81 (1 H, br. s), 4.15 (1 H, m), 3.48 (2 H, m), 2.92 (4 H, m), 1.79 (4 H, m), 1.62 (2 H, m) | 433 |

### Example 3

### Method B

### 4-(2-Hydroxy-2-phenyl-ethylamino)-2-oxo-1,2-dihydro-pyridine-3-carboxylic acid [4-(4-acetyl-piperazin-1-yl)-3-trifluoromethyl-phenyl]-amide

A mixture of 4-chloro-2-oxo-1,2-dihydro-pyridine-3-carboxylic acid [4-(4-acetyl-piperazin-1-yl)-3-trifluoromethyl-phenyl]-amide and 4-iodo-2-oxo-1,2-dihydropyridine-3-carboxylic acid [4-(4-acetyl-piperazin-1-yl)-3-trifluoromethyl-phenyl]-amide (0.15g) was added to a stirred solution of 2-hydroxy-2-phenyl-ethylamine (0.046g, 0.34mmol) and triethylamine (0.104ml, 0.75mmol) in acetonitrile (10ml) and the reaction was heated to refulx. After 20 hours the reaction was cooled to room temperature and concentrated under reduced pressure. The crude solid was dissolved in DCM and washed with water (20ml) and brine. The organic layer was concentrated under reduced pressure to give the crude product as oil (0.066g). This oil was dissolved in acetonitrile: water (1:1; 1ml) and purified by preparative HPLC (TFA) to give the desired compound as the TFA salt (5.0mg, 2.7%); Tr = 4.24 min, *m*/*z* (ES⁺) (M+H)⁺ 544; 1H NMR (400 MHz, DMSO-d6) δ ppm 13.31 (1 H, s), 11.20 (1 H, br s), 10.71 (1 H, t), 8.11 (1 H, d), 7.68 (1 H, dd), 7.54 (1 H, d), 7.45 (2 H, d), 7.39 - 7.24 (4 H, m), 6.11 (1 H, d), 5.79 (1 H, d), 4.85 - 4.77 (1 H, m), 3.58 - 3.49 (5 H, m), 3.35-3.31 (m, 1H), 2.80 (4 H, dt), 2.05 (3 H, s).

### Example 4

According to Method B described for Example 3 the following examples have been prepared:

| **Example** | **Systematic name** | **NMR (400 MHz, d6-DMSO):** δ | **MS (ES+): *m*/*z*** |
|---|---|---|---|
| **4a** | 4-[2-(3-Chlorophenyl)-ethylamino]-2-oxo-1,2-dihydro-pyridine-3-carboxylic acid [3-fluoro-4-(4-methyl-piperazin-1-yl)-phenyl] -amide | 13.11 (1 H, s), 11.24 (1 H, d), 10.54 (1 H, t), 9.62 (1 H, br. s.), 7.72 (1 H, dd), 7.45 - 7.42 (1 H, m), 7.39 - 7.32 (2 H, m), 7.32 - 7.26 (2 H, m), 7.17 - 7.12 (1H, m), 7.07 (1 H, t), 6.15 (1 H, d), 3.59 - 3.52 (2 H, m), 3.44 (4 H, br. s.), 3.23 (4 H, | 484 & 486 |
| **4b** | 4-[2-(3-Chlorophenyl)-ethylamino]-2-oxo-1,2-dihydro-pyridine-3-carboxylic acid [4-(4-methyl-piperazin-1-yl)-phenyl]-amide | 12.84 (1 H, s), 11.13 (1 H, d), 10.65 (1 H, t), 7.49 (2 H, d), 7.42 - 7.29 (5 H, m), 6.98 (2 H, d), 6.10 (1 H, d), 3.57 (2 H, q), 3.30 (4 H, m), 2.92 (2 H, t), 2.87 (3 H, s), 2.50 (4 H, m) | 466 & 468 |
| **4c** | 4-[(S)-2-Hydroxy-2-phenyl-ethylamino]-2-oxo-1,2-dihydro-pyridine-3-carboxylic acid [3-fluoro-4-(4-methyl-piperazin-1-yl)-phenyl]-amide | 12.93 (s, 1H), 11.10 (d, 1H), 10.71 (t, 1H), 7.70 (dd, 1H), 7.44 (m, 2H), 7.37-7.34 (m, 2H), 7.31-7.26 (m, 2H), 7.17 (m, 1H), 7.08 (t, 1H), 6.08 (d, 1H), 5.83 (d, 1H), 4.81 (dd, 1H), 3.53 (m, 1H), 3.49-3.41 (m, 4H), 3.38 (m, 1H), 3.02 - 2.96 (m, 4H), 2.87 (s, 3H) | 466 |
| **4d** | 4-[(S)-2-(3-Chlorophenyl)-2-hydroxy-ethylamino] -2-oxo-1,2-dihydro-pyridine-3-carboxylic acid [4-(4-acetyl-piperazin-1-yl)-3-trifluoromethyl-phenyl]-amide | 13.21 (1 H, s), 11.13 (1H, d), 10.65 (1H, t), 8.10 (1 H, s), 7.66 (1 H, d), 7.52 - 7.31 (6 H, m), 6.11 (1 H, d), 4.85 (1H, br. s), 4.14 (1 H, m), 3.55 (4 H, m), 3.41 (2 H, m), 2.81 (4 H, m), 2.05 (3H, s) | 578 & 580 |
| **4e** | 4-[(S)-2-Hydroxy-2-phenyl-ethylamino] -2-oxo-1,2-dihydro-pyridine-3-carboxylic acid (4-dimethylamino-phenyl)-amide | 12.96 (1 H, br. s.), 11.13 (1 H, d), 10.82 (1 H, t), 7.54 (2 H, d), 7.44 (2 H, d), 7.36 - 7.24 (4 H, m), 7.04 (2 H, br. s.), 6.07 (1 H, d), 4.79 (1 H, dd), 2.97 (6 H, s) | 393 |
| **4f** | 4-[2-(3-Chlorophenyl)-ethylamino] - 2-oxo-1,2-dihydro-pyridine-3-carboxylic acid (4-dimethylamino-phenyl)-amide | 12.80 (1 H, br. s.), 11.16 (1 H, d), 10.69 (1 H, t), 7.45 - 7.42 (3 H, m), 7.35 - 7.26 (4 H, m), 6.83 (2 H, br. s.), 6.12 (1 H, d), 3.55 - 3.50 (2 H, m), 2.91 - 2.88 (8 H, m) | 411 & 413 |
| **4g** | 2-Oxo-4-[(pyridin-2-ylmethyl)-amino] -1,2-dihydro-pyridine-3-carboxylic acid (4-dimethylamino-phenyl)-amide | 13.07 (1 H, br. s.), 11.31 (1 H, d), 11.08 (1 H, t), 8.62 (1 H, d), 7.90 (1 H, td), 7.62 (2 H, d), 7.46 - 7.35 (3 H, m), 7.21 - 7.20 (2 H, m), 6.05 (1 H, d), 4.72 (2 H, d), 3.04 (6 H, s) | 364 |
| **4h** | 4-[(S)-2-(3-Chlorophenyl)-2-hydroxy-ethylamino]-2-oxo-1,2-dihydro-pyridine-3-carboxylic acid (2,6-dimethyl-phenyl)-amide | 12.14 (s, 1H), 11.08 (s, 1H), 10.78 (t, 1H), 7.46 (s, 1H), 7.38- 7.28 (m, 4H), 7.13-7.03 (m, 3H), 6.05 (d, 1H), 5.80 (d, 1H), 4.79 (q, 1H), 3.52-3.47 (m, 1H), 3.38-3.31 (m, 1H), 2.22 (s, 6H) | 412,4 |
| **4i** | 4-[(S)-2-(3-Chlorophenyl)-2-hydroxy-ethylamino] -2-oxo-1,2-dihydro-pyridine-3-carboxylic acid [4-methoxy-3-(4-methyl-piperazin-1-yl)-phenyl]-amide | 12.84 (s, 1H), 11.08 (bs, 1H), 10.82 (t, 1H), 7.51 (s, 1H), 7.41 - 7.27 (m, 4H), 7.17-7.11 (m, 2H), 6.86 (d, 1H), 6.09 (d, 1H), 5.86 (bs, 1H), 4.82 (q, 1H), 3.76 (s, 3H), 3.56-3.50 (m, 2H), 3.02 - 2.90 (m, 4H), 2.55-2.41 (m, 4H), 2.46 (s, 3H) | 512,5 |
| **4j** | 4-[(S)-2-(3-Chlorophenyl)-2-hydroxy-ethylamino] -2-oxo-1,2-dihydro-pyridine-3-carboxylic acid (2,6-dimethoxy-phenyl)-amide | 11.63 (s, 1H), 11.07 (s, 1H), 10.91 (t, 1H), 7.53 (s, 1H), 7.45 - 7.32 (m, 4H), 7.24 (t, 1H), 6.74 (d, 2H), 6.10 (d, 1H), 5.87 (d, 1H), 4.82 (q, 1H), 3.78 (s, 6H), 3.60-3.52 (m, 1H), 3.41-3.37 (m, 1H) | 444,4 |
| **4k** | 4-[(S)-2-(3-Chlorophenyl)-2-hydroxy-ethylamino] -2-oxo-1,2-dihydro-pyridine-3-carboxylic acid (2-chloro-6-methyl-phenyl)-amide | 12.37 (s, 1H), 11.11 (bs, 1H), 10.69 (t, 1H), 7.46 (s, 1H), 7.38- 7.29 (m, 5H), 7.24 (d, 1H), 7.18 (t, 1H), 6.07 (d, 1H), 5.83 (d, 1H), 4.78 (q, 1H), 3.54-3.48 (m, 1H), 3.38-3.29 (m, 1H), 2.21 (s, 3H) | 432,4 |
| **4l** | 4-[(S)-2-(3-Chlorophenyl)-2-hydroxy-ethylamino] -2-oxo-1,2-dihydro-pyridine-3-carboxylic acid (2-methoxy-6-methyl-phenyl)-amide | 11.91 (s, 1H), 11.10 (t, 1H), 10.81 (t, 1H), 7.47 (s, 1H), 7.38- 7.27 (m, 4H), 7.11 (t, 1H), 6.87- 6.82 (m, 2H), 6.05 (d, 1H), 5.80 (bs, 1H), 4.78 (q, 1H), 3.73 (s, 3H), 3.54-3.47 (m, 1H), 3.37- 3.25 (m, 1H), 2.14 (s, 3H) | 428,5 |
| **4m** | 4-[(S)-2-(3-Chlorophenyl)-2-hydroxy-ethylamino]-2-oxo-1,2-dihydro-pyridine-3-carboxylic acid (3-morpholin-4-yl-phenyl)-amide | 12.98 (s, 1H), 11.11 (s, 1H), 10.78 (t, 1H), 7.52 (s, 1H), 7.41- 7.29 (m, 4H), 7.24 (s, 1H), 7.15 (t, 1H), 7.00 (d, 1H), 6.65 (dd, 1H), 6.10 (d, 1H), 5.87 (d, 1H), 4.85 (q, 1H), 4.06-4.00 (m, 4H), 3.76-3.74 (m, 1H), 3.57-3.51 (m, 1H), 3.11-3.08 (m, 4H | 469,2 |
| **4n** | 4-[(S)-2-(3-Chlorophenyl)-2-hydroxy-ethylamino]-2-oxo-1,2-dihydro-pyridine-3-carboxylic acid (4-morpholin-4-yl-phenyl)-amide | 12.83 (s, 1H), 11.10 (d, 1H), 10.86 (s, 1H), 7.51-7.26 (m, 7H), 6.91 (d, 2H), 6.08 (d, 1H), 5.88 (d, 1H), 4.82 (g, 1H), 3.74 (m, 4H), 3.54-3.51(m, 1H), 3.38-3.30 (m, 1H), 3.05 (m, 4H) | 469,5 |
| **4o** | 4-[(S)-2-(3-Chlorophenyl)-2-hydroxy-ethylamino]-2-oxo-1,2-dihydro-pyridine-3-carboxylic acid (4-carbamoyl-2,6-dimethyl-phenyl)-amide | 12.31 (s, 1H), 11.11(bs, 1H), 10.72 (s, 1H), 7.85 (s, 1H), 7.61 (s, 2H), 7.46 (s, 1H), 7.38 - 7.29 (m, 4H), 7.22 (s, 1H), 6.07 (d, 1H), 5.82 (d, 1H), 4.80 (q, 1H), 3.54-3.48 (m, 1H) 3.40-3.33 (m, 1H), 2.20 (s, 6H) | 455,4 |
| **4p** | 4-[(S)-2-(3-Chlorophenyl)-2-hydroxy-ethylamino]-2-oxo-1,2-dihydro-pyridine-3-carboxylic acid (3-methanesulfonyl-phenyl)-amide | 13.46 (s, 1H), 11.23 (bs, 1H), 10.70 (bs, 1H), 8.32 (s, 1H), 7.79 (bs, 1H), 7.59 (bs, 2H), 7.52 (s, 1H), 7.43-7.32 (m, 4H), 6.13 (d, 1H), 5.52 (d, 1H), 4.85 (q, 1H), 3.55 (m, 1H), 3.40-3.38 (m, 1H), 3.22 (s, 3H) | 462,5 |
| **4q** | 4-[(S)-2-(3-Chlorophenyl)-2-hydroxy-ethylamino] -2-oxo-1,2-dihydro-pyridine-3-carboxylic acid (3-methylsulfanyl-phenyl)-amide | 13.10 (s, 1H), 11.14 (bs, 1H), 10.75 (bs, 1H), 7.65 (s, 1H), 7.52 (s, 1H), 7.40-7.24 (m, 6H), 6.94 (s, 1H), 6.10 (d, 1H), 5.89 (d, 1H), 4.84 (q, 1H), 3.57-3.53 (m, 1H), 3.39- 3.37 (m, 1H), 2.48 (s, 3H) | 430,5 |
| **4r** | 4-((S)-2-Hydroxy-2-phenyl-ethylamino)-2-oxo-1,2-dihydro-pyridine-3-carboxylic acid (3-piperidin-1-ylmethyl-phenyl)-amide | 13.07 (s, 1H), 11.13 (bs, 1H), 10.81 (t, 1H), 7.50-7.26 (m, 7H), 6.97 (d, 1H), 6.08 (d,1H), 5.75 (d, 1H), 4.81-4.79 (q, 1H), 3.71- 3.67 (m, 1H), 3.53-3.46 (m, 1H), 2.37 (bs, 4H), 1.52-1.40 (m, 4H), 1.24 (m, 2H) | 447,3 |
| **4s** | 4-((S)-2-Hydroxy-2-phenyl-ethylamino)-2-oxo-1,2-dihydro-pyridine-3-carboxylic acid (3-pyrrolidin-1-ylmethyl-phenyl)-amide | 13.05 (s, 1H), 11.13 (bs, 1H), 10.82 (t, 1H), 7.53 (s, 1H), 7.48- 7.22 (m, 9H), 6.97 (d, 1H), 5.75 (m, 1H), 4.80 (q, 1H), 3.54 (s, 2H), 3.50 (m, 1H), 3.17 (m, 1H), 2.43 (m, 4H), 1.70 (s, 4H) | 433,2 |
| **4t** | 4-[(2-Hydroxy-2-phenyl-ethyl)-methyl-amino]-2-oxo-1,2-dihydro-pyridine-3-carboxylic acid [3-(4-methyl-piperazin-1-yl)-phenyl]-amide | 11.02 (d, 1H), 10.74 (m, 1H), 7.45-7.12 (m, 9H), 6.71 (m, 1H), 6.18 (d, 1H), 5.77 (bs, 1H), 4.81 (m, 1H), 3.75 (m, 2H), 3.43-3.35 (m, 4H), 2.96-2.87 (m, 7H), 2.78 (s, 3H) | 462,2 |
| **4u** | 4-(2-Hydroxy-2-phenyl-ethylamino)-2-oxo-1,2-dihydro-pyridine-3-carboxylic acid [3-(4-methyl-piperazin-1-yl)-phenyl]-amide | 12.98 (1H, s), 11.13 (1H, d), 10.76 (1H, t), 7.45 - 7.18 (8H, m), 7.12 (1H, m), 6.72 (1H, d), 6.08 (1H, d), 5.75 (1H, bs), 4.79 (1H, m), 3.81 (2H, m), 3.50 (2H, m), 3.38 (2H, m), 3.16 (2H, m), 2.96 (2H, m), 2.85 (3H, s) | 448 |
| **4v** | 2-Oxo-4-phenethylamino-1,2-dihydro-pyridine-3-carboxylic acid [3-(4-methyl-piperazin-1-yl)-phenyl]-amide | 13.01 (s, 1H), 11.18 (d, 1H), 10.62 (t, 1H), 7.39-7.15 (m, 8H), 7.05 (d, 1H), 6.71 (m, 1H), 6.12 (d, 1H), 3.81 (d, 2H), 3.54-3.47 (m, 4H), 3.17 (m, 2H), 2.97- 2.85 (m, 4H), 2.83 (s, 3H) | 432,1 |
| **4w** | 2-Oxo-4-(2-piperidin-1-yl-ethylamino)-1,2-dihydro-pyridine-3-carboxylic acid [3-(4-methyl-piperazin-1-yl)-phenyl]-amide | 13.03 (s, 1H), 11.37 (d, 1H), 10.62 (t, 1H), 7.45 (t, 1H), 7.22- 7.16 (m, 3H), 6.72 (m, 1H), 6.15 (d, 1H), 3.81 (m, 2H), 3.72 (m, 2H), 3.48-3.43 (m, 4H), 3.22 (m, 2H), 3.15 (m, 2H), 2.96- 2.88 (m, 4H), 2.83 (s, 3H), 1.83 (m, 2H), 1.67-1.61 (m, 3H), 1.38 (m, 1H) | 439,3 |
| **4x** | 4-(Methyl-phenethyl-amino)-2-oxo-1,2-dihydro-pyridine-3-carboxylic acid [3-(4-methyl-piperazin-1-yl)-phenyl]-amide | 11.00 (d, 1H), 10.57 (m, 1H), 7.45 (s, 1H), 7.30-7.12 (m, 8H), 6.69 (m, 1H), 6.05 (d, 1H), 3.75 (m, 2H), 3.55-3.48 (m, 4H), 3.17 (m, 2H), 2.96-2.85 (m, 7H), 2.83 (s, 3H) | 446,1 |
| **4y** | 4-[2-(3-Methoxy-phenyl)-ethylamino]-2-oxo-1,2-dihydro-pyridine-3-carboxylic acid [3-(4-methyl-piperazin-1-yl)-phenyl] -amide | 13.01 (s, 1H), 11.20 (d, 1H), 10.61 (t, 1H), 7.36 (m, 1H), 7.28-7.19 (m, 3H), 7.07 (m, 1H) 6.91-6.89 (m, 2H), 6.79 (m, 1H), 6.71 (m, 1H), 6.12 (m, 1H), 3.79-3.75 (m, 2H), 3.74 (s, 3H), 3.54-3.49 (m, 4H), 3.14 (m, 2H), 2.97-2.89 (m, 4H), 2.87 (s, 3H) | 462,1 |
| **4z** | 4-[(2-Hydroxy-2-phenyl-ethyl)-methyl-amino]-2-oxo-1,2-dihydro-pyridine-3-carboxylic acid [4-(4-methyl-piperazin-1-yl)-phenyl]-amide | 11.01 (d, 1H), 10.64 (m, 1H), 7.57-7.52 (m, 2H), 7.39-7.15 (m, 6H), 6.97-6.94 (m, 2H), 6.17 (d, 1H), 5.52 (bs, 1H), 4.86 (m, 1H), 3.75 (m, 2H), 3.49-3.41 (m, 4H), 2.97 (s, 3H), 2.95-2.84 (m, 4H), 2.82 (s, 3H) | 462,3 |
| **4aa** | 2-Oxo-4-(2-m-tolyl-ethylamino)-1,2-dihydro-pyridine-3-carboxylic acid [3-(4-methyl-piperazin-1-yl)-phenyl]-amide | 13.01 (s, 1H), 11.17 (d, 1H), 10.60 (t, 1H), 7.35 (m, 1H), 7.26-7.02 (m, 7H), 6.70 (m, 1H), 6.11 (m, 1H), 3.82-3.79 (m, 2H), 3.54-3.49 (m, 4H), 3.18-3.14 (m, 2H), 2.99-2.89 (m, 4H), 2.83 (s, 3H), 2.28 (s, 3H) | 446,2 |
| **4ab** | 4-[2-(2-Methoxy-phenyl)-ethylamino]-2-oxo-1,2-dihydro-pyridine-3-carboxylic acid [3-(4-methyl-piperazin-1-yl)-phenyl]-amide | 13.02 (s, 1H), 11.17 (d, 1H), 10.59 (t, 1H), 7.39 (m, 1H), 7.29-7.17 (m, 4H), 7.04 (m, 1H) 6.91-6.87 (m, 2H), 6.70 (m, 1H), 6.13 (m, 1H), 3.83 (s, 3H), 3.82-3.79 (m, 2H), 3.54-3.43 (m, 4H), 3.19-3.17 (m, 2H), 2.98- 2.87 (m, 4H), 2.84 (s, 3H) | 462,1 |
| **4ac** | 4-[2-(3H-Imidazol-4-yl)-ethylamino]-2-oxo-1,2-dihydro-pyridine-3-carboxylic acid [3-(4-methyl-piperazin-1-yl)-phenyl]-amide | 13.02 (s, 1H), 11.29 (m, 1H), 10.64 (m, 1H), 9.87 (bs, 1H), 9.01 (s, 1H), 7.51 (s, 1H), 7.39 (m, 1H), 7.19-7.14 (m, 3H), 6.71 (m, 1H), 6.13 (m, 1H), 3.76-3.72 (m, 2H), 3.58-3.56 (m, 2H), 3.48-3.44 (m, 2H), 3.17- 3.12 (m, 2H), 2.97-2.93 (m, 4H), 2.86 (s, 3H) | 422,2 |
| **4ad** | 4-[2-(4-Methoxy-phenyl)-ethylamino]-2-oxo-1,2-dihydro-pyridine-3-carboxylic acid [3-(4-methyl-piperazin-1-yl)-phenyl]-amide | 13.01 (s, 1H), 11.16 (d, 1H), 10.6 (t, 1H), 7.34 (m, 1H), 7.25- 7.19 (m, 4H), 7.08 (m, 1H) 6.91-6.87 (m, 2H), 6.70 (m, 1H), 6.13 (m, 1H), 3.83 (s, 3H), 3.82-3.79 (m, 2H), 3.54-3.43 (m, 4H), 3.19-3.17 (m, 2H), 2.98- 2.87 (m, 4H), 2.84 (s, 3H) | 462,1 |
| **4ae** | 4-[2-(1H-Indol-3-yl)-ethylamino]-2-oxo-1,2-dihydro-pyridine-3-carboxylic acid [3-(4-methyl-piperazin-1-yl)-phenyl]-amide | 13.02 (s, 1H), 11.15 (m, 1H), 10.88 (m, 1H), 10.62 (m, 1H), 7.59 (d, 1H), 7.37-7.35 (m, 2H), 7.24-7.17 (m, 3H), 7.10-7.05 (m, 2H), 6.98 (t, 1H), 6.70 (d, 1H), 6.11 (d, 1H), 3.82-3.74 (m, 2H), 3.57-3.48 (m, 4H), 3.18-3.14 (m, 2H), 3.03-2.91 (m, 4H), 2.87 (s, 3H) | 471,5 |

### Example 5

### Method C

### 2-Oxo-4-[(pyridin-2-ylmethyl)-amino]-1,2-dihydro-pyridine-3-carboxylic acid (3-morpholin-4-yl-phenyl)-amide

2-Methoxy-N-(3-morpholin-4-yl-phenyl)-4- [(pyridin-2-ylmethyl)-amino]-nicotinamide (54 mg, 0.13 mmol) is dissolved in 2 ml dioxane, treated with conc. HCl (0.2 ml) and heated to reflux (110°C). Based on reaction control, the reaction mixture is poured onto CH2Cl2 after a few hours and adjusted to alkaline pH with saturated aqueous NaHCO3-solution. The final product is isolated by extraction with CH2Cl2, the organic phases are dried and the solvent is evaporated. LCMS purification yields 31 mg (60 %) of a white powder; *m*/*z* (ES⁺) (M+H)⁺ 406,5; 1H NMR (400 MHz, DMSO-d6) δ ppm 13.00 (s, 1H), 11.21 (bs, 1H), 11.10 (t, 1H), 8.57 (d, 1H), 7.80 (ddd, 1H), 7.38-7.30 (m, 3H), 7.25 (s, 1H), 7.16 (t, 1H), 7.02 (d, 1H), 6.65 (dd, 1H), 6.03 (d, 1H), 4.68 (d, 2H), 3.75-3.72 (m, 4H), 3.10-3.08 (m, 4H).

### Example 6

According to Method C described for Example 5 the following examples have been prepared:

| **Example** | **Systematic name** | **NMR (400 MHz, d6-DMSO):** δ | **MS (ES+): *m*/*z*** |
|---|---|---|---|
| **6a** | 2-Oxo-4- [(pyridin-2-ylmethyl)-amino] -1,2-dihydro-pyridine-3-carboxylic acid (2,6-dimethyl-phenyl)-amide | 12.16 (s, 1H), 11.20 (s, 1H), 11.12 (t, 1H), 9.55 (d, 1H), 7.79 (ddd, 1H), 7.36-7.28 (m, 3H), 7.10-7.04 (m, 3H), 6.05 (d, 1H), 4.62 (d, 2H), 2.17 (s, 6H) | 349,4 |
| **6b** | 2-Oxo-4- [(pyridin-2-ylmethyl) -amino]-1,2-dihydro-pyridine-3-carboxylic acid [4-methoxy-3-(4-methyl-piperazin-1-yl)-phenyl]-amide | 12.86 (s, 1H), 11.20 (s, 1H), 11.12 (t, 1H), 8.57 (d, 1H), 7.80 (ddd, 1H),7.38-7.29 (m, 3H), 7.18-7.12 (m, 2H), 6.87 (d, 1H), 6.02 (d, 1H), 4,.67 (d, 2H), 3.76 (s, 3H), 3.02-2.89 (m, 4H), 2.48- 2.41 (m, 4H), 2.22 (s, 3H) | 449,5 |
| **6c** | 2-Oxo-4-[(pyridin-2-ylmethyl)-amino]-1,2-dihydro-pyridine-3-carboxylic acid (2-methoxy-6-methyl-phenyl)-amide | 11.92 (s, 1H), 11.13 (m, 2H), 8.54 (d,1H), 7.79 (ddd, 1H), 7.38-7.27 (m, 3H), 7.12 (t, 1H), 6.89-6.80 (m, 2H), 6.03 (d, 1H), 4.62 (d, 2H), 3.73 (s, 3H), 2.15 (s, 3H) | 365,5 |
| **6d** | 2-Oxo-4-[(pyridin-2-ylmethyl)-amino]-1,2-dihydro-pyridine-3carboxylic acid (2,6-dimethoxy-phenyl)-amide | 11.59 (s, 1H), 11.15 (m, 2H), 8.54 (d, 1H), 7.79 (t, 1H), 7.35- 7.28 (m, 3H), 7.19 (t, 1H), 6.68 (d, 2H), 6.02 (d, 1H), 4.61 (d, 2H), 3.72 (s, 6H) | 380,8 |
| **6e** | 2-Oxo-4-[(pyridin-2-ylmethyl)-amino]-1,2-dihydro-pyridine-3-carboxylic acid (2-chloro-6-methyl-phenyl)-amide | 12.38 (s, 1H), 11.23 (m, 2H), 8.55 (d, 1H), 7.80 (ddd, 1H), 7.40-7.17 (m, 6H), 6.06 (d, 1H), 4.64 (d, 2H), 2.22 (s, 3H) | 369,3 |
| **6f** | 2-Oxo-4-phenethylamino-1,2-dihydro-pyridine-3-carboxylic acid (3-morpholin-4-yl-phenyl)-amide | 12.96 (s, 1H), 11.14 (s, 1H), 10.64 (t, 1H), 7.34-7.20 (m, 7H), 7.15 (m, 1H), 6.96 (d, 1H), 6.64 (dd, 1H), 6.11 (d, 1H), 3.76-3.73 (m, 4H), 3.54 (q, 2H), 3.10-3.07 (m, 4H), 2.89 (t, 2H) | 419,4 |
| **6g** | 2-Oxo-4-(2-phenyl-propylamino)-1,2-dihydro-pyridine-3-carboxylic acid (3-morpholin-4-yl-phenyl)-amide | 12.93 (s, 1H), 11.11 (bs, 1H), 10.64 (t, 1H), 7.32-7.12 (m, 8H), 6.95 (d, 1H), 6.63 (dd, 1H), 6.09 (d, 1H), 3.76-3.74 (m, 4H), 3.53-3.43 (m, 2H), 3.09-3.02 (m, 5H), 1.29 (d, 3H) | 433,6 |
| **6h** | 2-Oxo-4-(2-pyridin-2-yl-ethylamino)-1,2-dihydro-pyridine-3-carboxylic acid (3-morpholin-4-yl-phenyl)-amide | 12.94 (s, 1H), 11.13 (bs, 1H), 10.65 (t, 1H), 8.53 (d, 1H), 7.72 (ddd, 1H), 7.34 (d, 2H), 7.25- 7.22 (m, 2H), 7.14 (t, 1H), 6.95 (d, 1H), 6.64 (dd, 1H), 6.11 (d, 1H), 3.76-3.73 (m, 4H), 3.69 (q, 2H), 3.09-3.03 (m, 6H) | 420,4 |
| **6i** | 2-Oxo-4-phenethylamino-1,2-dihydro-pyridine-3-carboxylic acid [4-methoxy-3-(4-methyl-piperazin-1-yl)-phenyl]-amide | 12.81 (s, 1H), 11.12 (d, 1H), 10.67 (t, 1H), 7.34-7.11 (m, 8H), 6.86 (d, 1H), 6.10 (d, 1H), 3.75 (s, 3H), 3.53 (q, 2H), 2.96-2.87 (m, 6H), 2.52-2.46 (m, 4H), 2.23 (s, 3H) | 462,4 |
| **6j** | 2-Oxo-4-(2-phenyl-propylamino)-1,2-dihydro-pyridine-3-carboxylic acid [4-methoxy-3-(4-methyl-piperazin-1-yl)-phenyl]-amide | 12.78 (s, 1H), 11.08 (d, 1H), 10.68 (t, 1H), 7.32-7.31 (m, 5H), 7.29-7.20 (m, 1H), 7.12-7.06 (m, 2H), 6.85 (d, 1H), 6.08 (m, 1H), 3.75 (s, 3H), 3.50-3.44 (m, 2H), 3.07-2.96 (m, 5H), 2.49-2.46 (m, 4H), 2.22 (s, 3H), 1.29 (d, 3H) | 476,4 |
| **6k** | 2-Oxo-4-(2-pyridin-2-yl-ethylamino)-1,2-dihydro-pyridine-3-carboxylic acid [4-methoxy-3-(4-methyl-piperazin-1-yl)-phenyl]-amide | 12.79 (s, 1H), 11.12 (d, 1H), 10.68 (t, 1H), 8.53 (d, 1H), 7.72 (ddd, 1H), 7.33 (t, 2H), 7.25- 7.22 (m, 1H), 7.12-7.10 (m, 2H), 6.85 (d, 1H), 6.10 (d, 1H), 3.75 (s, 3H), 3.68 (q, 2H), 3.05 (t, 2H), 2.96 (m, 4H), 2.46 (m, 4H), 2.22 (s, 3H) | 463,4 |
| **6l** | 4-(1-Methyl-piperidin-4-ylamino)-2-oxo-1,2-dihydro-pyridine-3-carboxylic acid [4-methoxy-3-(4-methyl-piperazin-1-yl)-phenyl] -amide | 12.84 (s, 1H), 11.10 (s, 1H), 10.72 (d, 1H), 7.31 (t, 1H), 7.21 (dd, 1H), 7.00 (d, 1H), 6.86 (d, 1H), 6.09 (d, 1H), 3.79 (s, 3H), 3.75-6.67 (m, 1H), 3.50-3.46 (m, 2H), 2.96 (m, 4H), 2.66-2.63 (m, 2H), 2.45 (m, 4H), 2.22 (s, 3H), 2.18 (s, 3H), 2.15-2.06 (m, 2H), 1.53-1.45 (m, 2H) | 455,4 |
| **6m** | 4-Cyclohexylamino-2-oxo-1,2-dihydro-pyridine-3-carboxylic acid [4-methoxy-3-(4-methyl-piperazin-1-yl)-phenyl]-amide | 12.85 (s, 1H), 11.07 (bs, 1H), 10.72 (d, 1H), 7.30 (t, 1H), 7.19 (dd, 1H), 7.02 (d, 1H), 6.86 (d, 1H), 6.08 (d, 1H), 3.75 (s, 3H), 3.51 (m, 1H), 2.96 (m, 4H), 2.45 (m, 4H), 2.22 (s, 3H), 1.90-1.74 (m, 2H), 1.67-1.61 (m, 2H), 1.60-1.55 (m, 1H), 1.42-1.19 (m, 5H) | 440,5 |
| **6n** | 2-Oxo-4-(2-pyrrolidin-1-yl-ethylamino)-1,2-dihydro-pyridine-3-carboxylic acid [4-methoxy-3-(4-methyl-piperazin-1-yl)-phenyl]-amide | 12.79 (s, 1H), 11.09 (bs, 1H), 10.64 (t, 1H), 7.33 (d, 1H), 7.14 (dd, 1H), 7.10 (d, 1H), 6.86 (d, 1H), 6.05 (d, 1H), 3.71 (s, 3H), 3.40-3.12 (m, 6H), 2.96 (m, 4H), 2.65 (t, 2H), 2.51 (m, 4H), 2.22 (s, 3H), 1.74-1.69 (m, 4H) | 455,3 |
| **6o** | 4-[2-(3-Chloro-phenyl)-ethylamino] -2-oxo-1,2-dihydro-pyridine-3-carboxylic acid [4-methoxy-3-(4-methyl-piperazin-1-yl)-phenyl]-amide | 12.82 (s, 1H), 11.12 (bs, 1H), 10.65 (t, 1H), 7.42 (s, 1H), 7.36- 7.27 (m, 4H), 7.14-7.10 (m, 2H), 6.85 (d, 1H), 6.12 (d, 1H), 3.75 (s, 3H), 3.53 (q, 2H), 2.96- 2.89 (m, 6H), 2.46 (m, 4H), 2.22 (s, 3H) | 496,4 |
| **6p** | 4-Benzenesulfonylamino-2-oxo-1,2-dihydro-pyridine-3-carboxylic acid [4-methoxy-3-(4-methyl-piperazin-1-yl)-phenyl] -amide | 12.83 (s, 1H), 12.33 (s, 1H), 11.85 (s, 1H), 7.99-7.91 (m, 2H), 7.74-7.61 (m, 4H), 7.20 (d, 1H), 7.12 (s, 1H), 6.93 (d, 1H), 6.62 (d, 1H), 3.78 (s, 3H), 3.00 (m, 4H), 2.48 (m, 4H), 2.33 (s, 3H) | 498,2 |
| **6q** | 2-Oxo-4-[(pyridin-2-ylmethyl)-amino] -1,2-dihydro-pyridine-3-carboxylic acid (4-carbamoyl-2,6-dimethyl-phenyl)-amide | 12.32 (s, 1H), 11.23 (bs, 1H), 11.10 (s, 1H), 8.54 (d, 1H), 7.85- 7.77 (m, 2H), 7.61 (s, 2H), 7.34- 7.22 (m, 4H), 6.06 (d, 1H), 4.64 (d, 2H), 2.22 (s, 6H) | 392,4 |
| **6r** | 2-Oxo-4-[(pyridin-2-ylmethyl)-amino]-1,2-dihydro-pyridine-3-carboxylic acid (4-morpholin-4-yl-phenyl)-amide | 12.84 (s, 1H), 11.21 (s, 1H), 11.16 (t, 1H), 8.58 (d, 1H), 7.82 (t, 1H), 7.47 (d, 2H), 7.38-7.30 (m, 3H), 6.90 (d, 2H), 6.04 (d, 1H), 4.66 (d, 2H), 3.77-3.72 (m, 4H), 3.06-3.04 (m, 4H) | 406,4 |
| **6s** | 2-Oxo-4-[(pyridin-2-ylmethyl)-amino]-1,2-dihydro-pyridine-3-carboxylic acid (3-methylsulfanyl-phenyl)-amide | 13.12 (s, 1H), 11.26 (bs, 1H), 11.05 (t, 1H), 8.58 (d, 1H), 7.82- 7.78 (m, 1H), 7.64 (s, 1H), 7.39- 7.25 (m, 5H), 6.95-6.94 (m, 1H), 6.05 (d, 1H), 4.68 (d, 2H), 2.49 (s, 3H) | 367,5 |
| **6t** | 2-Oxo-4-phenethylamino-1,2- dihydro-pyridine-3-carboxylic acid (3-piperidin-1-ylmethyl-phenyl)-amide | 13.03 (s, 1H), 11.17 (bs, 1H), 10.64 (t, 1H), 7.47 (m, 2H), 7.32-7.21 (m, 7H), 6.95 (d, 1H), 6.12 (d, 1H), 3.57-3.52 (m, 2H), 3.43 (s, 2H), 2.92-2.89 (m, 2H), 2.32 (m, 4H), 1.50-1.48 (m, 4H) 1.39 (m, 2H) | 431,4 |
| **6u** | 2-Oxo-4-(2-phenyl-propylamino)-1,2-dihydro-pyridine-3-carboxylic acid (3-piperidin-1-ylmethyl-phenyl)-amide | 13.01 (s, 1H), 11.15 (bs, 1H), 10.65 (t, 1H), 7.47-7.42 (m, 2H), 7.31 m, 5H), 7.25-7.21 (m, 2H), 6.99 (d, 1H), 6.09 (d, 1H), 4.07 (bs, 2H), 3.49-3.43 (m, 2H), 3.38 (s, 2H), 3.08- 2.99 (m, 1H), 2.32 (m, 5H), 1.50.- 1.48 (m, 4H), 1.40 (d, 2H) | 445,3 |
| **6v** | 2-Oxo-4-(2-phenyl-propylamino)-1,2-dihydro-pyridine-3-carboxylic acid (3-pyrrolidin-1-ylmethyl-phenyl)-amide | 13.02 (s, 1H), 11.15 (bs, 1H), 10.65 (s, 1H), 7.46 (t, 2H), 7.33 (t, 4H), 7.23-7.16 (m, 2H), 6.85 (d, 1H), 6.81-6.79 (m, 1H), 6.09 (d, 1H), 3.77 (s, 2H), 3.55-3.48 (t, 2H), 3.06 (m, 1H), 2.50-2.44 (m, 4H), 1.70 (s, 3H), 1.30 (d, 4H) | 431,3 |
| **6w** | 2-Oxo-4-phenethylamino-1,2-dihydro-pyridine-3-carboxylic acid (3-pyrrolidin-1 -ylmethyl-phenyl)-amide | 13.03 (s, 1H), 10.64 (t, 1H), 7.51 (t, 1H), 7.45 (d, 1H), 7.35- 7.31(m, 5H), 7.23 (m, 3H), 6.97 (d, 1H), 6.12 (d, 1H), 3.54 (s, 4H), 2.92-2.89 (m, 2H), 2.43 (s, 4H), 1.70 (bs, 4H) | 417,3 |
| **6x** | 2-Oxo-4-phenethylamino-1,2-dihydro-pyridine-3-carboxylic acid (3-diethylaminomethyl-4-hydroxy-phenyl)-amide | 12.74 (s, 1H), 10.69 (m, 1H), 7.31-7.21 (m, 10H), 6.63 (d, 1H), 6.09 (d, 1H), 3.71 (s, 2H), 3.53-3.51 (m, 2H), 2.91-2.87 (m, 2H), 2.58-2.55 (m, 4H), 1.05- 1.01(m, 6H) | 435,5 |
| **6y** | 4-Ethylamino-2-oxo-1,2-dihydro-pyridine-3-carboxylic acid [4-methoxy-3-(4-methyl-piperazin-1-yl)-phenyl]-amide | 12.84 (s, 1H), 11.11 (d, 1H), 10.51 (t, 1H), 7.33 (t, 1H), 7.14 (dd, 1H), 7.10 (d, 1H), 6.86 (d, 1H), 6.04 (d, 1H), 3.75 (s, 3H), 3.41-3.28 (m, 2H), 2.96-2.87 (m, 4H), 2.52-2.38 (m, 4H), 2.22 (s, 3H), 1.20 (t, 3H) | 386,2 |
| **6z** | 4-Isopropylamino-2-oxo-1,2-dihydro-pyridine-3-carboxylic acid [4-methoxy-3-(4-methyl-piperazin-1-yl)-phenyl]-amide | 12.86 (s, 1H), 11.09 (d, 1H), 10.61 (d, 1H), 7.32 (t, 1H), 7.19 (dd, 1H), 7.03 (d, 1H), 6.85 (d, 1H), 6.06 (d, 1H), 3.85-3.79 (m, 1H), 3.75 (s, 3H), 3.02-2.88 (m, 4H), 2.52-2.39 (m, 4H), 2.22 (s, 3H), 1.22 (d, 6H) | 400,2 |
| **6aa** | 2-Oxo-4-(2-pyridin-3-yl-ethylamino)-1,2-dihydro-pyridine-3-carboxylic acid [4-methoxy-3-(4-methyl-piperazin-1-yl)-phenyl]-amide | 12.81 (s, 1H), 11.12 (bs, 1H), 10.69 (t, 1H), 8.52 (s, 1H), 8.43 (d, 1H), 7.73 (d, 1H), 7.32 (t, 2H), 7.12 (t, 2H), 6.86 (d, 1H), 6.12 (d, 1H), 3.75 (s, 3H), 3.57 (q, 2H), 2.96-2.90 (m, 6H), 2.46 (m, 4H), 2.22 (s, 3H) | 463,2 |
| **6ab** | 2-Oxo-4-(2-pyridin-4-yl-ethylamino)-1,2-dihydro-pyridine-3-carboxylic acid [4-methoxy-3-(4-methyl-piperazin-1-yl)-phenyl]-amide | 12.81 (s, 1H), 11.14 (d, 1H), 10.67 (t, 1H), 8.48 (d, 2H), 7.35- 7.32 (m, 3H), 7.12-7.10 (m, 2H), 6.87-6.84 (m, 1H), 6.12 (d, 1H), 3.75 (s, 3H), 3.58 (q, 2H), 2.96-2.90 (m, 6H), 2.46 (m, 4H), 2.22 (s, 3H) | 463,2 |
| **6ac** | 4-(Methyl-phenethyl-amino)-2-oxo-1,2-dihydro-pyridine-3-carboxylic acid [4-methoxy-3-(4-methyl-piperazin-1-yl)-phenyl] -amide | 10.92 (bs, 1H), 10.33 (s, 1H), 7.32-7.14 (m, 8H), 6.84 (d, 1H), 6.03 (d, 1H), 3.74 (s, 3H), 3.49 (t, 2H), 2.99-2.92 (m, 7H), 2.85 (t, 2H), 2.45 (m, 4H), 2.21 (s, 3H) | 476,2 |
| **6ad** | 2-Oxo-4-phenethylamino-1,2-dihydro-pyridine-3-carboxylic acid [4-(4-methyl-piperazin-1-yl)-phenyl]-amide | 12,27 (br s, 1H); 11,08 (br s, 2H); 7,52 (d, 2H); 7,38-7,30 (m, 2H); 7,29-7,20 (m, not separated from CHCl3); 7,19-7,05 (m, 1H); 6,93 (d, 2H); 5,92 (s and br s, together 2H); 3,52 (q, 2H); 3,24 (s, 4H); 2,99 (t, 2H); 2,75 (s, 4H); 2,42 (s, 3H) | 432,4 |
| **6ae** | 2-Oxo-4-(2-phenyl-propylamino)-1,2-dihydro-pyridine-3-carboxylic acid [4-(4-methyl-piperazin-1-yl)-phenyl] -amide | 12,30 (br s, 1H); 11,13 (br s, 2H); 7,51 (d, 2H); 7,40-7,30 (m, 2H); 7,30-7,21 (m, not separated from CHCl3); 7,19-7,03 (m, 1H); 6,94 (d, 2H); 5,88 (br s, 1H); 4,15 (br s, 1H); 3,54-3,42 (m, 1H); 3,41-3,32 (m, 1H); 3,22 (s, 4H); 3,10 (br q, 1H); 2,65 (s, 4H); 2,39 (s, 3H); 1,41 (d, 3H) | 446,4 |

## Claims

1. A compound of formula I, 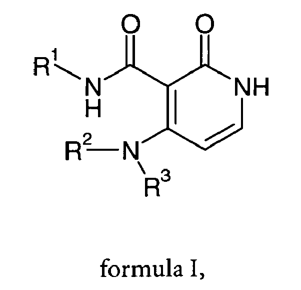 wherein
R¹ is phenyl, which is unsubstituted or substituted one or several times, independently by alkyl, -OH, -O-alkyl, -S-alkyl, -S(O)₂- alkyl, halogen, -NRR', -CH₂-NRR', trifluoromethyl, trifluoromethoxy, heterocyclyl which is unsubstituted or substituted once or twice by alkyl or acetyl, -CH₂-heterocyclyl, - C(O)-NRR', -C(O)-NH-CH₂-phenyl-R", -C(O)-NH-phenyl- R";
R² is hydrogen or (C₁-C₃)alkyl;
R³ is -X-R⁴;
R⁴ is a) (C₁-C₄)alkyl; b) (C₃-C₆)cycloalkyl; c) a 5 to 7 membered heterocycle, which is unsubstituted or substituted once or twice by alkyl; d) phenyl, wherein the phenyl is unsubstituted or substituted one to three times by halogen, alkyl, -O-CH₃, -N(CH₃)₂, trifluoromethyl or trifluoromethoxy; or e) heteroaryl;
X is (C₁-C₄)alkylene, -S(O)₂-, or a single bond, wherein the (C₁-C₄)alkylene is unsubstituted or substituted once or twice by hydroxy, alkyl or halogen;
R and R' represent independently of each other hydrogen or (C₁-C₃)alkyl;
and all pharmaceutically acceptable salts thereof.

2. The compounds according to claim 1, **characterized in that**
R¹ is phenyl, which is unsubstituted or substituted one to three times independently by alkyl, -OH, -O-CH₃, -S-CH₃, -S(O)₂- CH₃, chlorine, fluorine, -NRR', -CH₂-NRR', trifluoromethyl, - CH₂-pprolyl, -CH₂-piperidinyl, -C(O)-NRR', or heterocyclyl selected from pyrrolidinyl, piperidinyl, morpholino, piperazinyl, methyl-piperazinyl, or acetyl-piperazinyl;
R² is hydrogen or methyl; preferably hydrogen;
R⁴ is a) (C₁-C₃)alkyl; b) cyclohexyl; c) a 5 to 7 membered heterocycle selected from pyrrolidinyl, piperidinyl or methyl-piperidinyl; d) phenyl, wherein the phenyl is unsubstituted or substituted once by chlorine, alkyl, -O-CH₃, -N(CH₃)₂, or trifluoromethyl; or e) heteroaryl selected from pyridyl, indolyl or imidazolyl; and
X is (C₁-C₄)alkylene, -S(O)₂-, or a single bond, wherein the (C₁-C₄)alkylene is unsubstituted or substituted once by hydroxy or methyl.

3. The compounds according to any one of claims 1 to 2, **characterized in that**
R⁴ is a) (C₁-C₃)alkyl; or
b) cyclohexyl.

4. The compounds according to any one of claims 1 to 2, **characterized in that**
R⁴ is a 5 to 7 membered heterocycle selected from pyrrolidinyl, piperidinyl or methyl-piperidinyl.

5. The compounds according to any one of claims 1 to 2, **characterized in that**
R⁴ is phenyl, wherein the phenyl is unsubstituted or substituted once by chlorine, alkyl, -O-CH₃, -N(CH₃)₂, or trifluoromethyl.

6. The compounds according to any one of claims 1 to 2, **characterized in that**
R⁴ is heteroaryl selected from pyridyl, indolyl or imidazolyl.

7. The compounds according to claim 1, selected from the group consisting of:
4-Ethylamino-2-oxo-1,2-dihydro-pyridine-3-carboxylic acid [4-methoxy-3-(4-methyl-piperazin-1-yl)-phenyl] -amide;
4-Isopropylamino-2-oxo-1,2-dihydro-pyridine-3-carboxylic acid [4-methoxy-3-(4-methyl-piperazin-1-yl)-phenyl]-amide;
4-Cyclohexylamino-2-oxo-1,2-dihydro-pyridine-3-carboxylic acid [4-methoxy-3-(4-methyl-piperazin-1-yl)-phenyl]-amide;
2-Oxo-4-(2-piperidin-1-yl-ethylamino)-1,2-dihydro-pyridine-3-carboxylic acid [3-(4-methyl-piperazin-1-yl)-phenyl]-amide;
4-(1-Methyl-piperidin-4-ylamino)-2-oxo-1,2-dihydro-pyridine-3-carboxylic acid [4-methoxy-3-(4-methyl-piperazin-1-yl)-phenyl]-amide;
2-Oxo-4-(2-pyrrolidin-1-yl-ethylamino)-1,2-dihydro-pyridine-3-carboxylic acid [4-methoxy-3-(4-methyl-piperazin-1-yl)-phenyl]-amide;
4-[2-(3-Chloro-phenyl)-ethylamino]-2-oxo-1,2-dihydro-pyridine-3-carboxylic acid (3-fluoro-4-morpholin-4-yl-phenyl)-amide;
4-((S)-2-Hydroxy-2-phenyl-ethylamino)-2-oxo-1,2-dihydro-pyridine-3-carboxylic acid (3-fluoro-4-morpholin-4-yl-phenyl)-amide;
4-[(S)-2-(3-Chloro-phenyl)-2-hydroxy-ethylamino]-2-oxo-1,2-dihydropyridine-3-carboxylic acid (3-fluoro-4-morpholin-4-yl-phenyl)-amide;
4-((S)-2-Hydroxy-2-phenyl-ethylamino)-2-oxo-1,2-dihydro-pyridine-3-carboxylic acid (3-pyrrolidin-1-yl-phenyl)-amide;
4-[2-(3-Chloro-phenyl)-ethylamino]-2-oxo-1,2-dihydro-pyridine-3-carboxylic acid (3-pyrrolidin-1-yl-phenyl)-amide;
4-[(S)-2-(3-Chloro-phenyl)-2-hydroxy-ethylamino]-2-oxo-1,2-dihydropyridine-3-carboxylic acid (4-dimethylamino-phenyl)-amide;
4-[2-(3-Chloro-phenyl)-2-hydroxy-ethylamino]-2-oxo-1,2-dihydropyridine-3-carboxylic acid (4-piperidin-1-yl-phenyl)-amide;
4-[(S)-2-(3-Chloro-phenyl)-2-hydroxy-ethylamino]-2-oxo-1,2-dihydropyridine-3-carboxylic acid (4-pyrrolidin-1-yl-3-trifluoromethyl-phenyl)-amide;
4-[2-(3-Chloro-phenyl)-ethylamino]-2-oxo-1,2-dihydro-pyridine-3-carboxylic acid (4-pyrrolidin-1-yl-3-trifluoromethyl-phenyl)-amide;
4-[(S)-2-Hydroxy-2-phenyl-ethylamino]-2-oxo-1,2-dihydro-pyridine-3-carboxylic acid (4-pyrrolidin-1-yl-3-trifluoromethyl-phenyl)-amide;
4-[2-(3-Chloro-phenyl)-ethylamino]-2-oxo-1,2-dihydro-pyridine-3-carboxylic acid (3-piperidin-1-yl-phenyl)-amide;
4-[(S)-2-(3-Chloro-phenyl)-2-hydroxy-ethylamino]-2-oxo-1,2-dihydropyridine-3-carboxylic acid [4-(4-methyl-piperazin-1-yl)-phenyl]-amide;
4-[(S)-2-Hydroxy-2-phenyl-ethylamino]-2-oxo-1,2-dihydro-pyridine-3-carboxylic acid [4-(4-methyl-piperazin-1-yl)-phenyl]-amide;
4-[(S)-2-(3-Chloro-phenyl)-2-hydroxy-ethylamino]-2-oxo-1,2-dihydropyridine-3-carboxylic acid (3-piperidin-1-yl-phenyl)-amide;
4-[(S)-2-Hydroxy-2-phenyl-ethylamino]-2-oxo-1,2-dihydro-pyridine-3-carboxylic acid (3-dimethylamino-phenyl)-amide;
4-[2-(3-Chloro-phenyl)-ethylamino]-2-oxo-1,2-dihydro-pyridine-3-carboxylic acid (3-dimethylamino-phenyl)-amide;
4-[(S)-2-(3-Chloro-phenyl)-2-hydroxy-ethylamino]-2-oxo-1,2-dihydropyridine-3-carboxylic acid (3-dimethylamino-phenyl)-amide;
4-[(S)-2-Hydroxy-2-phenyl-ethylamino]-2-oxo-1,2-dihydro-pyridine-3-carboxylic acid (3-piperidin-1-yl-phenyl)-amide;
4-[(S)-2-Hydroxy-2-phenyl-ethylamino]-2-oxo-1,2-dihydro-pyridine-3-carboxylic acid [4-(4-acetyl-piperazin-1-yl)-3-trifluoromethyl-phenyl]-amide;
4-[2-(3-Chloro-phenyl)-ethylamino]-2-oxo-1,2-dihydro-pyridine-3-carboxylic acid [3-fluoro-4-(4-methyl-piperazin-1-yl)-phenyl]-amide;
2-Oxo-4-(2-m-tolyl-ethylamino)-1,2-dihydro-pyridine-3-carboxylic acid [3-(4-methyl-piperazin-1-yl)-phenyl]-amide;
4-[2-(2-Methoxy-phenyl)-ethylamino]-2-oxo-1,2-dihydro-pyridine-3-carboxylic acid [3-(4-methyl-piperazin-1-yl)-phenyl]-amide;
4-[2-(4-Methoxy-phenyl)-ethylamino]-2-oxo-1,2-dihydro-pyridine-3-carboxylic acid [3-(4-methyl-piperazin-1-yl)-phenyl]-amide;
4-[2-(3-Chloro-phenyl)-ethylamino]-2-oxo-1,2-dihydro-pyridine-3-carboxylic acid [4-(4-methyl-piperazin-1-yl)-phenyl]-amide;
4-[(S)-2-Hydroxy-2-phenyl-ethylamino]-2-oxo-1,2-dihydro-pyridine-3-carboxylic acid [3-fluoro-4-(4-methyl-piperazin-1-yl)-phenyl]-amide;
4-[(S)-2-(3-Chloro-phenyl)-2-hydroxy-ethylamino]-2-oxo-1,2-dihydropyridine-3-carboxylic acid [4-(4-acetyl-piperazin-1-yl)-3-trifluoromethylphenyl]-amide;
4-[(S)-2-Hydroxy-2-phenyl-ethylamino]-2-oxo-1,2-dihydro-pyridine-3-carboxylic acid (4-dimethylamino-phenyl)-amide;
4-[2-(3-Chloro-phenyl)-ethylamino]-2-oxo-1,2-dihydro-pyridine-3-carboxylic acid (4-dimethylamino-phenyl)-amide;
4-[(S)-2-(3-Chloro-phenyl)-2-hydroxy-ethylamino]-2-oxo-1,2-dihydropyridine-3-carboxylic acid (2,6-dimethyl-phenyl)-amide;
4-[(S)-2-(3-Chloro-phenyl)-2-hydroxy-ethylamino]-2-oxo-1,2-dihydropyridine-3-carboxylic acid [4-methoxy-3-(4-methyl-piperazin-1-yl)-phenyl] -amide;
4-[(S)-2-(3-Chloro-phenyl)-2-hydroxy-ethylamino]-2-oxo-1,2-dihydropyridine-3-carboxylic acid (2,6-dimethoxy-phenyl)-amide;
4-[(S)-2-(3-Chloro-phenyl)-2-hydroxy-ethylamino]-2-oxo-1,2-dihydropyridine-3-carboxylic acid (2-chloro-6-methyl-phenyl)-amide;
4-[(S)-2-(3-Chloro-phenyl)-2-hydroxy-ethylamino]-2-oxo-1,2-dihydropyridine-3-carboxylic acid (2-methoxy-6-methyl-phenyl)-amide;
4-[(S)-2-(3-Chloro-phenyl)-2-hydroxy-ethylamino]-2-oxo-1,2-dihydropyridine-3-carboxylic acid (3-morpliolin-4-yl-phenyl)-amide;
4-[(S)-2-(3-Chloro-phenyl)-2-hydroxy-ethylamino]-2-oxo-1,2-dihydropyridine-3-carboxylic acid (4-morpholin-4-yl-phenyl)-amide;
4-[(S)-2-(3-Chloro-phenyl)-2-hydroxy-ethylamino]-2-oxo-1,2-dihydropyridine-3-carboxylic acid (4-carbamoyl-2,6-dimethyl-phenyl)-amide;
4-[(S)-2-(3-Chloro-phenyl)-2-hydroxy-ethylamino]-2-oxo-1,2-dihydropyridine-3-carboxylic acid (3-methanesulfonyl-phenyl)-amide;
4-[(S)-2-(3-Chloro-phenyl)-2-hydroxy-ethylamino]-2-oxo-1,2-dihydropyridine-3-carboxylic acid (3-methylsulfanyl-phenyl)-amide;
4-((S)-2-Hydroxy-2-phenyl-ethylamino)-2-oxo-1,2-dihydro-pyridine-3-carboxylic acid (3-piperidin-1-ylmethyl-phenyl)-amide;
4-((S)-2-Hydroxy-2-phenyl-ethylamino)-2-oxo-1,2-dihydro-pyridine-3-carboxylic acid (3-pyrrolidin-1-ylmethyl-phenyl)-amide;
4-[(2-Hydroxy-2-phenyl-ethyl)-methyl-amino]-2-oxo-1,2-dihydropyridine-3-carboxylic acid [3-(4-methyl-piperazin-1-yl)-phenyl]-amide;
4-(2-Hydroxy-2-phenyl-ethylamino)-2-oxo-1,2-dihydro-pyridine-3-carboxylic acid [3-(4-methyl-piperazin-1-yl)-phenyl]-amide;
2-Oxo-4-phenethylamino-1,2-dihydro-pyridine-3-carboxylic acid [3-(4-methyl-piperazin-1-yl)-phenyl]-amide;
4-(Methyl-phenethyl-amino)-2-oxo-1,2-dihydro-pyridine-3-carboxylic acid [3-(4-methyl-piperazin-1-yl)-phenyl]-amide;
4-[2-(3-Methoxy-phenyl)-ethylamino]-2-oxo-1,2-dihydro-pyridine-3-carboxylic acid [3-(4-methyl-piperazin-1-yl)-phenyl]-amide;
4-[(2-Hydroxy-2-phenyl-ethyl)-methyl-amino]-2-oxo-1,2-dihydropyridine-3-carboxylic acid [4-(4-methyl-piperazin-1-yl)-phenyl]-amide;
2-Oxo-4-phenethylamino-1,2-dihydro-pyridine-3-carboxylic acid (3-morpholin-4-yl-phenyl)-amide;
2-Oxo-4-(2-phenyl-propylamino)-1,2-dihydro-pyridine-3-carboxylic acid (3-morpholin-4-yl-phenyl)-amide;
2-Oxo-4-phenethylamino-1,2-dihydro-pyridine-3-carboxylic acid [4-methoxy-3-(4-methyl-piperazin-1-yl)-phenyl]-amide;
2-Oxo-4-(2-phenyl-propylamino)-1,2-dihydro-pyridine-3-carboxylic acid [4-methoxy-3-(4-methyl-piperazin-1-yl)-phenyl]-amide;
4- [2-(3-Chloro-phenyl)-ethylamino] -2-oxo-1,2-dihydro-pyridine-3-carboxylic acid [4-methoxy-3-(4-methyl-piperazin-1-yl)-phenyl]-amide;
4-Benzenesulfonylamino-2-oxo-1,2-dihydro-pyridine-3-carboxylic acid [4-methoxy-3-(4-methyl-piperazin-1-yl)-phenyl]-amide;
2-Oxo-4-phenethylamino-1,2-dihydro-pyridine-3-carboxylic acid (3-piperidin-1-ylmethyl-phenyl)-amide;
2-Oxo-4-(2-phenyl-propylamino)-1,2-dihydro-pyridine-3-carboxylic acid (3-piperidin-1-ylmethyl-phenyl)-amide;
2-Oxo-4-(2-phenyl-propylamino)-1,2-dihydro-pyridine-3-carboxylic acid (3-pyrrolidin-1-ylmethyl-phenyl)-amide;
2-Oxo-4-phenethylamino-1,2-dihydro-pyridine-3-carboxylic acid (3-pyrrolidin-1-ylmethyl-phenyl)-amide;
2-Oxo-4-phenethylamino-1,2-dihydro-pyridine-3-carboxylic acid (3-diethylaminomethyl-4-hydroxy-phenyl)-amide;
4-(Methyl-phenethyl-amino)-2-oxo-1,2-dihydro-pyridine-3-carboxylic acid [4-methoxy-3-(4-methyl-piperazin-1-yl)-phenyl]-amide;
2-Oxo-4-phenethylamino-1,2-dihydro-pyridine-3-carboxylic acid [4-(4-methyl-piperazin-1-yl)-phenyl]-amide;
2-Oxo-4-(2-phenyl-propylamino)-1,2-dihydro-pyridine-3-carboxylic acid [4-(4-methyl-piperazin-1-yl)-phenyl]-amide;
2-Oxo-4-[(pyridin-2-ylmethyl)-amino]-1,2-dihydro-pyridine-3-carboxylic acid (3-fluoro-4-morpholin-4-yl-phenyl)-amide;
2-Oxo-4-[(pyridin-2-ylmethyl)-amino]-1,2-dihydro-pyridine-3-carboxylic acid (3-pyrrolidin-1-yl-phenyl)-amide;
2-Oxo-4-[(pyridin-2-ylmethyl)-amino]-1,2-dihydro-pyridine-3-carboxylic acid (4-pyrrolidin-1-yl-3-trifluoromethyl-phenyl)-amide;
2-Oxo-4-[(pyridin-2-ylmethyl)-amino]-1,2-dihydro-pyridine-3-carboxylic acid [3-fluoro-4-(4-methyl-piperazin-1-yl)-phenyl]-amide;
2-Oxo-4-[(pyridin-2-ylmethyl)-amino]-1,2-dihydro-pyridine-3-carboxylic acid (3-piperidin-1-yl-phenyl)-amide;
2-Oxo-4-[(pyridin-2-ylmethyl)-amino]-1,2-dihydro-pyridine-3-carboxylic acid [4-(4-methyl-piperazin-1-yl)-phenyl]-amide;
2-Oxo-4-[(pyridin-2-ylmethyl)-amino]-1,2-dihydro-pyridine-3-carboxylic acid (3-dimethylamino-phenyl)-amide;
4-[2-(3H-Imidazol-4-yl)-ethylamino]-2-oxo-1,2-dihydro-pyridine-3-carboxylic acid [3-(4-methyl-piperazin-1-yl)-phenyl]-amide;
4-[2-(1H-Indol-3-yl)-ethylamino]-2-oxo-1,2-dihydro-pyridine-3-carboxylic acid [3-(4-methyl-piperazin-1-yl)-phenyl]-amide;
2-Oxo-4-[(pyridin-2-ylmethyl)-amino]-1,2-dihydro-pyridine-3-carboxylic acid (4-dimethylamino-phenyl)-amide;
2-Oxo-4-[(pyridin-2-ylmethyl)-amino]-1,2-dihydro-pyridine-3-carboxylic acid (3-morpholin-4-yl-phenyl)-amide;
2-Oxo-4-[(pyridin-2-ylmethyl)-amino]-1,2-dihydro-pyridine-3-carboxylic acid (2,6-dimethyl-phenyl)-amide;
2-Oxo-4-[(pyridin-2-ylmethyl)-amino]-1,2-dihydro-pyridine-3-carboxylic acid [4-methoxy-3-(4-methyl-piperazin-1-yl)-phenyl]-amide;
2-Oxo-4-[(pyridin-2-ylmethyl)-amino]-1,2-dihydro-pyridine-3-carboxylic acid (2-methoxy-6-methyl-phenyl)-amide;
2-Oxo-4-[(pyridin-2-ylmethyl)-amino]-1,2-dihydro-pyridine-3-carboxylic acid (2,6-dimethoxy-phenyl)-amide;
2-Oxo-4- [(pyridin-2-ylmethyl)-amino] -1,2-dihydro-pyridine-3-carboxylic acid (2-chloro-6-methyl-phenyl)-amide;
2-Oxo-4-(2-pyridin-2-yl-ethylamino)-1,2-dihydro-pyridine-3-carboxylic acid (3-morpholin-4-yl-phenyl)-amide;
2-Oxo-4-(2-pyridin-2-yl-ethylamino)-1,2-dihydro-pyridine-3-carboxylic acid [4-methoxy-3-(4-methyl-piperazin-1-yl)-phenyl]-amide;
2-Oxo-4-[(pyridin-2-ylmethyl)-amino]-1,2-dihydro-pyridine-3-carboxylic acid (4-carbamoyl-2,6-dimethyl-phenyl)-amide;
2-Oxo-4-[(pyridin-2-ylmethyl)-amino]-1,2-dihydro-pyridine-3-carboxylic acid (4-morpholin-4-yl-phenyl)-amide;
2-Oxo-4- [(pyridin-2-ylmethyl)-amino]-1,2-dihydro-pyridine-3-carboxylic acid (3-methylsulfanyl-phenyl)-amide;
2-Oxo-4-(2-pyridin-3-yl-ethylamino)-1,2-dihydro-pyridine-3-carboxylic acid [4-methoxy-3-(4-methyl-piperazin-1-yl)-phenyl]-amide; and
2-Oxo-4-(2-pyridin-4-yl-ethylamino)-1,2-dihydro-pyridine-3-carboxylic acid [4-methoxy-3-(4-methyl-piperazin-1-yl)-phenyl]-amide.

8. A process for the preparation of the compounds of formula I comprising the steps of:
a) reacting a compound of formula III, 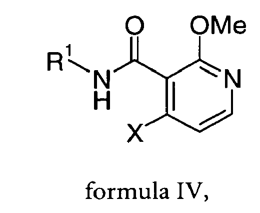 wherein R¹ has the meaning of formula I as defined above in claim 1 and X represents a halogen selected from chlorine, bromine and iodine,
with a compound of formula VII 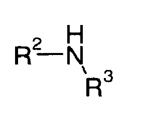 wherein R¹ and R² have the meaning of formula I as defined above in claim 1,
b) cleavage of the methoxy group of the pyridine residue,
wherein such cleavage of the methoxy group of the pyridine can also be performed before step a)
to give the compounds of formula I, 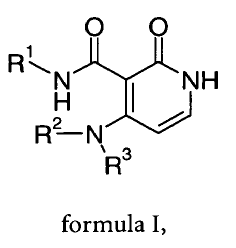 wherein R¹, R² and R³ have the meaning of formula I as defined above in claim 1.

9. A pharmaceutical composition, containing one or more compounds according to claims 1 to 7 as active ingredients together with pharmaceutically acceptable carriers.

10. Use of one or more compounds according to claims 1 to 7 for the treatment of cancer.
